Europäisches Patentamt
European Patent Office
Office européen des brevets

(19)

(11) Veröffentlichungsnummer: **0 239 528**
**A1**

(12)

# EUROPÄISCHE PATENTANMELDUNG

(21) Anmeldenummer: **87810109.6**

(22) Anmeldetag: **26.02.87**

(51) Int. Cl.⁴: **C 07 D 493/22**
C 07 F 7/04, A 61 K 31/35
//(C07D493/22,313:00,311:00,
311:00,307:00)

(30) Priorität: **05.03.86 CH 895/86**

(43) Veröffentlichungstag der Anmeldung:
**30.09.87 Patentblatt 87/40**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Maienfisch, Peter, Dr.**
**Traugott-Meyer-Strasse 5**
**CH-4147 Aesch (CH)**

(54) **13Beta-Carbamoyloxy-milbemycinderivate zur Bekämpfung von tier- und pflanzenparasitären Schädlingen.**

(57) Es werden neue 13β-Carbamoyloxy-milbemycine der Formel I

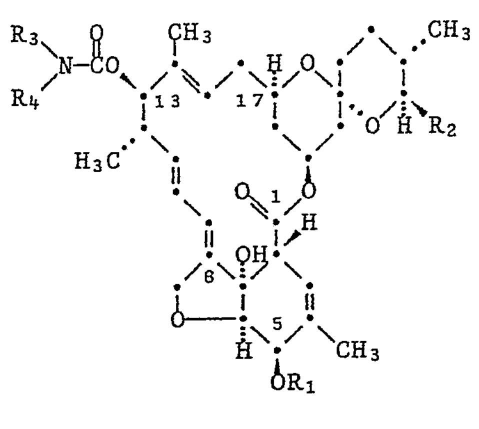

(I)

in welcher
$R_1$ Wasserstoff, eine Silyl- oder Acylgruppe,
$R_2$ Methyl, Ethyl, Isopropyl oder sek.Butyl,
$R_3$ eine unsubstituierte oder substituierte Gruppe ausgewählt aus der Reihe geradkettiges oder verzweigtes $C_1$-$C_{18}$-Alkyl, $C_3$-$C_{10}$-Cycloalkyl, $C_2$-$C_6$-Alkenyl, $C_3$-$C_{10}$-Cycloalkenyl, Phenyl und Naphthyl, und
$R_4$ Wasserstoff oder einen unter $R_3$ angegebenen Rest bedeuten, beschrieben, die sich als Wirkstoffe für Pestizide eignen und, die aus den zugrundeliegenden 13β-Hydroxymilbemycinen durch Carbamoylierung zugänglich sind.

EP 0 239 528 A1

**Beschreibung**

13β-Carbamoyloxy-milbemycin-derivate zur Bekämpfung von tier- und pflanzenparasitären Schädlingen

Die vorliegende Erfindung betrifft neue 13β-Carbamoyloxy-milbemycin-Derivate der Formel I, ihre Herstellung und ihre Verwendung zur Bekämpfung von Schädlingen, ferner Schädlingsbekämpfungsmittel, die mindestens eine dieser Verbindungen als Wirkstoff enthalten.

Die neuen Verbindungen entsprechen der allgemeinen Formel I

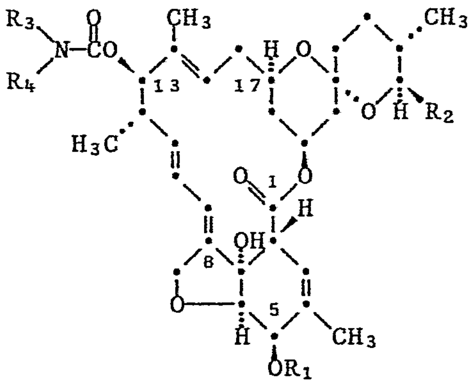

(I)

in welcher

$R_1$ Wasserstoff, eine Silyl- oder Acylgruppe,

$R_2$ Methyl, Ethyl, Isopropyl oder sek.Butyl,

$R_3$ eine unsubstituierte oder substituierte Gruppe ausgewählt aus der Reihe geradkettiges oder verzweigtes $C_1$-$C_{18}$-Alkyl, $C_3$-$C_{10}$-Cycloalkyl, $C_2$-$C_6$-Alkenyl, $C_3$-$C_{10}$-Cycloalkenyl, Phenyl und Naphthyl, und

$R_4$ Wasserstoff oder einen unter $R_3$ angegebenen Rest bedeuten.

Unter den vorgenannten Bedeutungen für $R_3$ und $R_4$ sind als bevorzugt anzusehen: $C_1$-$C_8$-Alkyl, $C_3$-$C_6$-Cycloalkyl, $C_2$-$C_6$-Alkenyl und $C_5$-$C_7$-Cycloalkyl.

Als Substituenten der Alkyl-, Cycloalkyl-, Alkenyl- und Cycloalkenyl-Gruppen kommen beispielsweise 1 bis 7 Halogenatome oder 1 bis 6 $C_1$-$C_6$-Alkoxygruppen, eine oder mehrere Acyloxygruppen, eine unsubstituerte oder substituierte Phenyl- oder Naphthylgruppe und als Substituenten der Phenyl- oder Naphthylgruppe 1 bis 3 Substituenten ausgewählt aus der Reihe bestehend aus Halogenatomen, $C_1$-$C_6$-Alkyl, $C_1$-$C_6$-Alkoxy, $C_1$-$C_4$-Alkylthio, $C_1$-$C_4$-Haloalkyl oder Nitro in Betracht. Bei den Cycloalkyl-und Cycloalkenylgruppen können als Substituenten auch $C_1$-$C_4$-Alkylgruppen vorliegen. Bei substituerten Gruppen können unterschiedliche Substituenten nebeneinander vorliegen. Naphthyl steht für α- und β-Naphthyl.

Unter dem Begriff Alkyl als Substituent oder Teil eines Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl, Nonyl oder Decyl, sowie die Isomeren, wie beispielsweise Isopropyl, Isobutyl, tert.-Butyl oder Isopentyl, Haloalkyl steht für einen einfach bis perhalogenierten Alkylsubstituenten, wie beispielsweise $CHCl_2$, $CHF_2$, $CH_2Cl$, $CCl_3$, $CF_3$, $CH_2F$, $CH_2CH_2Cl$, $CHBr_2$, vorzugsweise $CF_3$. Unter Halogen soll hier und im folgenden Fluor, Chlor, Brom oder Jod, vorzugsweise Fluor, Chlor oder Brom, verstanden werden. Als cycloaliphatische Gruppen kommen mono-bis tetracyclische Gruppen in Betracht, wie beispielsweise Cyclopropyl, Cyclobutyl, Cyclopentyl, Cyclohexyl, Decalin, Hydrindan, Bicycloheptan, Bicyclooctan, Norbornan, Bornan oder Adamantyl. Diese cycloaliphatischen Gruppen sind vorzugsweise unsubstituiert oder durch Methyl ein- oder mehrfach substituert. Alkenyl steht für einen mindestens durch eine C=C-Doppelbindung charakterisierten aliphatischen, acyclischen Kohlenwasserstoffrest, wie beispielsweise für Vinyl, Propenyl-(1), Allyl, Butenyl-(1), Butenyl-(2) oder Butenyl-(3). Ein durch Halogen substituiertes Alkenyl bedeutet dementsprechend einen derartigen Alkenylrest, der ein- oder mehrfach halogeniert ist. Cycloalkenyl steht z.B. für eine der genannten cycloaliphatischen Gruppen, die jedoch eine oder mehrere C=C-Doppelbindungen enthält. Ein durch Alkoxy substituiertes Alkyl steht für eine unverzweigte oder verzweigte, durch ein Sauerstoffatom unterbrochene Alkylgruppe, beispielsweise für $CH_2OCH_3$, $CH_2CH_2OCH_3$, $CH_2CH(CH_3)OCH_3$, $CH_2OC_2H_5$, $C(CH_3)_2OCH_3$, $CH_2OC_3H_7$-i oder $CH_2CH_2CH_2OCH_3$.

Als substituiertes Phenyl kommen beispielsweise 2,4-Dichlorphenyl, 2,5-Dimethoxyphenyl, p-Bromphenyl, 2,6-Xylyl, 3-Nitrophenyl, 4-Fluor-3-nitrophenyl, 4-Chlor-2-methylphenyl, 4-Methyl-2-methoxyphenyl, 2,6-Dimethylphenyl oder p-Methylthiophenyl in Betracht.

Verbindungen der Formel I, worin $R_1$ Wasserstoff bedeutet, sind bevorzugt. Acyl- und Silylgruppen als $R_1$ sind im allgemeinen als Schutzgruppen aufzufassen. Geeignete Acylgruppen sind beispielsweise die Reste $R_5$-C(O)-, wobie $R_5$ für $C_1$-$C_{10}$-Alkyl, $C_1$-$C_{10}$-Haloalkyl oder einen unsubstituierten oder durch Substituenten der Gruppe bestehend aus Halogen, $C_1$-$C_3$-Alkyl, $C_1$-$C_3$-Haloalkyl, $C_1$-$C_3$-Alkoxy, $C_1$-$C_3$-Haloalkoxy, Cyano und Nitro substituierten Rest aus der Gruppe Phenyl und Benzyl steht und vorzugsweise $C_1$-$C_6$-Alkyl,

$C_1$-$C_6$-Haloalkyl oder unsubstituiertes oder durch Halogen, $C_1$-$C_3$-Alkyl, $CF_3$ oder Nitro substituiertes Phenyl bedeutet. Besonders erwähnenswert ist die Acetylgruppe. Als Acylverbindung ist beispielsweise 5-O-Acetyl-13β-N-Methylcarbamoyloxy-milbemycin $A_4$ zu nennen. Als geeignete Silylgruppen für $R_1$ kommt der Rest $-Si(R_6)(R_7)(R_8)$ in Frage, wobei $R_6$, $R_7$ und $R_8$ vorzugsweise unabhängig voneinander für $C_1$-$C_6$-Alkyl, Benzyl oder Phenyl stehen und beispielsweise eine der Gruppen Trimethylsilyl, Thexyldimethylsilyl, Diphenyl-tert.butylsilyl, bis(Isopropyl)methylsilyl oder Triphenylsilyl und vorzugsweise tert.Butyldimethylsilyl bilden.

Verbindungen, worin $R_2$ sek.Butyl darstellt, sollen hier und im folgenden gleichfalls zu den Milbemycin-Derivaten gerechnet werden, obwohl sie nach der üblichen Systematik von Avermectin-Derivaten abgeleitet sind. Avermectin-Aglykone (mit einer OH-Gruppe in 13α-Position) lassen sich jedoch gemäss US-PS 4,173,571 in Milbemycin-Homologe überführen.

In natürlich vorkommenden Milbemycinen ($R_1=H$; $R_2=CH_3$, $C_2H_5$ oder iso $C_3H_7$) ist die 13-Position anstelle der 13β-Carbamoyloxy-gruppe der erfindungsgemässen Verbindungen der Formel I ausschliesslich mit Wasserstoff besetzt, wie die nachstehende Formel zeigt:

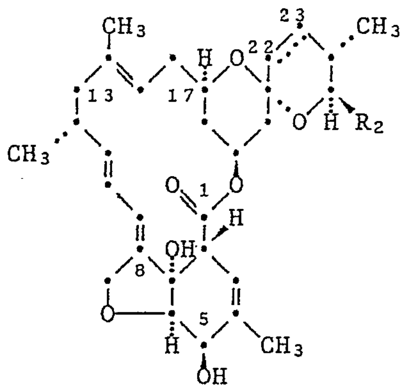

$R_2$ = $CH_3$ Milbemycin $A_3$
$R_2$ = $C_2H_5$ Milbemycin $A_4$
$R_2$ = iso$C_3H_7$ Milbemycin D
$R_2$ = sec.$C_4H_9$ 13-Deoxi-22,23-dihydro-C-076-Bla-aglycon.

Bei Avermectinen dagegen steht in der 13-Position ein α-L-Oleandrosyl-α-L-oleandrose-Rest, der über Sauerstoff in α-Konfiguration mit dem Makrolid-Molekül verknüpft ist. Avermectine unterscheiden sich strukturell ausserdem durch eine 23-OH-Gruppe oder $\Delta^{22,23}$-Doppelbindung und der Regel durch einen Substituenten $R_2$ = sek.$C_4H_9$ von den Milbemycinen. Durch Hydrolyse des Zucker-Restes der Avermectine gelangt man leicht zu den entsprechenden Avermectin-Aglykonen, die eine in Nachbarstellung zu einer C=C-Doppelbindung befindliche 13α-Hydroxy-Gruppe besitzen. Die Avermectin-Aglykone sind, wie vorstehend angegeben, in die Milbemycin- Homologen umwandelbar. Bei den Milbemycin-Derivaten der vorliegenden Anmeldung liegt die $\Delta^{22,23}$-Doppelbindung stets in hydrierter Form vor und der Substituent in 13-Position ist immer β-ständig.

Folgende Untergruppen von Verbindungen der Formel I sind auf Grund ihrer ausgeprägten parasitiziden und insektiziden Wirkung besonders bevorzugt:

Gruppe Ia: Verbindungen der Formel I, worin
$R_1$ Wasserstoff darstellt,
$R_2$ für Methyl, Ethyl, Isopropyl oder sek.Butyl steht,
$R_3$ unsubstituiertes oder durch Halogen, $C_1$-$C_4$-Alkoxy, Acyloxy oder eine unsubstituiertes oder durch Halogen, $C_1$-$C_2$-Alkyl, $C_1$-$C_2$-Alkoxy, $C_1$-$C_2$-Haloalkyl oder Nitro substituierte Phenyl- oder Naphthylgruppe substituiertes $C_1$-$C_{18}$-Alkyl, unsubstituiertes oder durch Halogen und/oder $C_1$-$C_4$-Alkyl substituiertes $C_3$-$C_{10}$-Cycloalkyl, unsubstituiertes oder durch Halogen substituiertes $C_2$-$C_6$-Alkenyl, unsubstituiertes oder durch Halogen und/oder $C_1$-$C_4$-Alkyl substituiertes $C_3$-$C_{10}$-Cycloalkenyl darstellt, und
$R_4$ für Wasserstoff steht.

Gruppe Ib: Verbindungen der Formel I, worin
$R_1$ Wasserstoff darstellt,
$R_2$ für Methyl, Ethyl, Isopropyl oder sek.Butyl steht, und
$R_3$ unsubstituiertes oder durch Halogen, Phenyl oder Naphthyl substituiertes $C_1$-$C_8$-Alkyl, $C_5$-$C_7$-Cycloalkyl, $C_2$-$C_6$-Alkenyl und $C_5$-$C_7$-Cycloalkenyl darstellt, und
$R_4$ für Wasserstoff steht.

Gruppe Ic: Verbindungen der Formel I, worin
$R_1$ Wasserstoff darstellt,
$R_2$ für Methyl, Ethyl, Isopropyl oder sek.Butyl steht,
$R_3$ unsubstituiertes oder durch Halogen, $C_1$-$C_6$-Alkyl, $C_1$-$C_6$-Alkoxy, $C_1$-$C_4$-Alkylthio, $C_1$-$C_4$-Haloalkyl oder Nitro substituiertes Phenyl oder Naphthyl darstellt, und
$R_4$ für Wasserstoff steht.

Gruppe Id: Verbindungen der Formel I, worin
$R_1$ Wasserstoff darstellt,
$R_2$ für Methyl, Ethyl, Isopropyl oder sek.Butyl steht, und
$R_3$ unsubstituiertes oder durch Brom, Chlor, Fluor, $C_1$-$C_2$-Alkyl, $C_1$-$C_2$-Alkoxy, $C_1$-$C_2$-Haloalkyl oder Nitro substituiertes Phenyl oder Naphthyl darstellt, und
$R_4$ für Wasserstoff steht.

Gruppe Ie: Verbindungen der Formel I, worin
$R_1$ Wasserstoff darstellt,
$R_2$ für Methyl, Ethyl, Isopropyl oder sek. Butyl steht, und
$R_3$ unsubstituiertes oder durch Brom, Chlor, Fluor, $C_1$-$C_2$-Alkyl, $C_1$-$C_2$-Alkoxy, Trifluormethyl oder Nitro substituiertes Phenyl oder Naphthyl darstellt.

Gruppe If: Verbindungen der Formel I, worin
$R_1$ Wasserstoff darstellt,
$R_2$ für Methyl, Ethyl, Isopropyl oder sek.Butyl steht, und
$R_3$ unsubstituiertes oder durch Halogen, $C_1$-$C_4$-Alkoxy, oder $C_1$-$C_4$-Acyloxy substituiertes $C_1$-$C_8$-Alkyl, unsubstituiertes oder durch Halogen, $C_1$-$C_6$-Alkyl, $C_1$-$C_4$-Alkylthio, $C_1$-$C_4$-Haloalkyl oder Nitro substituiertes Phenyl darstellt, und
$R_4$ einen unter $R_3$ angegebenen Rest bedeutet.

Gruppe Ig: Verbindungen der Formel I, worin
$R_1$ Wasserstoff darstellt,
$R_2$ für Methyl, Ethyl, Isopropyl oder sek.Butyl steht, und
$R_3$ $C_1$-$C_8$-Alkyl, oder Phenyl darstellt, und
$R_4$ einen unter $R_3$ angegebenen Rest bedeutet.

Besonders bevorzugte 5-Hydroxy-Derivate der Formel I sind z.B.:
13β-N-Methylcarbamoyloxy-milbemycin D,
13β-N-Methylcarbamoyloxy-milbemycin $A_3$,
13β-N-Methylcarbamoyloxy-milbemycin $A_4$,
13β-N-tert.-Butylcarbamoyloxy-milbemycin D,
13β-N-tert.-Butylcarbamoyloxy-milbemycin $A_3$,
13β-N-tert.-Butylcarbamoyloxy-milbemycin $A_4$,
13β-N-Phenylcarbamoyloxy-milbemycin D,
13β-N-Phenylcarbamoyloxy-milbemycin $A_3$,
13β-N-Phenylcarbamoyloxy-milbemycin $A_4$,
13β-N,N-Dimethylcarbamoyloxy-milbemycin D,
13β-N,N-Dimethylcarbamoyloxy-milbemycin $A_3$,
13β-N,N-Dimethylcarbamoyloxy-milbemycin $A_4$,
13β-N,N-Diisopropylcarbamoyloxy-milbemycin D,
13β-N,N-Diisopropylcarbamoyloxy-milbemycin $A_3$,
13β-N,N-Diisopropylcarbamoyloxy-milbemycin $A_4$.

Bevorzugte, an der 5-Hydroxygruppe geschützte Verbindungen der Formel I ($R_1 \neq H$) sind beispielsweise:
5-O-tert.-Butyldimethylsilyl-13β-N-methylylcarbamoyloxy-milbemycin D,
5-O-tert.-Butyldimethylsilyl-13β-N-methylylcarbamoyloxy-milbemycin $A_3$,
5-O-tert.-Butyldimethylsilyl-13β-N-methylylcarbamoyloxy-milbemycin $A_4$,
5-O-tert.-Butyldimethylsilyl-13β-N-tert.-butylcarbamoyloxy-milbemycin D,
5-O-tert.-Butyldimethylsilyl-13β-N-tert.-butylcarbamoyloxy-milbemycin $A_3$,
5-O-tert.-Butyldimethylsilyl-13β-N-tert.-butylcarbamoyloxy-milbemycin $A_4$,
5-O-tert.-Butyldimethylsilyl-13β-N-phenylcarbamoyloxy-milbemycin D,
5-O-tert.-Butyldimethylsilyl-13β-N-phenylcarbamoyloxy-milbemycin $A_3$,
5-O-tert.-Butyldimethylsilyl-13β-N-phenylcarbamoyloxy-milbemycin $A_4$,
5-O-tert.-Butyldimethylsilyl-13β-N,N-dimethylcarbamoyloxy-milbemycin D,
5-O-tert.-Butyldimethylsilyl-13β-N,N-dimethylcarbamoyloxy-milbemycin $A_3$,
5-O-tert.-Butyldimethylsilyl-13β-N,N-dimethylcarbamoyloxy-milbemycin $A_4$,
5-O-tert.-Butyldimethylsilyl-13β-N,N-diisopropylcarbamoyloxy-milbemycin D,
5-O-tert.-Butyldimethylsilyl-13β-N,N-diisopropylcarbamoyloxy-milbemycin $A_3$,
5-O-tert.-Butyldimethylsilyl-13β-N,N-diisopropylcarbamoyloxy-milbemycin $A_4$.

Bemerkenswert sind ferner folgende Verbindungen der Formel I:
5-O-Acetyl-13β-N-methylcarbamoyloxy-milbemycin $A_4$,
13β-N,N-Diphenylcarbamoyloxy-milbemycin $A_4$.

Die vorliegende Erfindung betrifft auch Herstellungsverfahren, die es erlauben, in der 13-Position von Milbemycin- oder 13-Deoxi-22,23-dihydro-Avermectinaglykon-Derivaten eine β-Carbamoyloxy-Gruppe gezielt einzuführen und damit zu den hochwirksamen, neuen Parasitiziden und Insektiziden der Formel I zu gelangen, die ihrerseits auch für weitere Derivatisierungen verwendet werden können. Die erfindungsgemässe Herstellung der Verbindungen der Formel I wird im Einzelnen wie folgt durchgeführt:

A. In den Fällen, in denen eine Verbindung der Formel I hergestellt werden soll, worin $R_4$ für Wasserstoff steht, wird ein Allylalkohol der Formel II

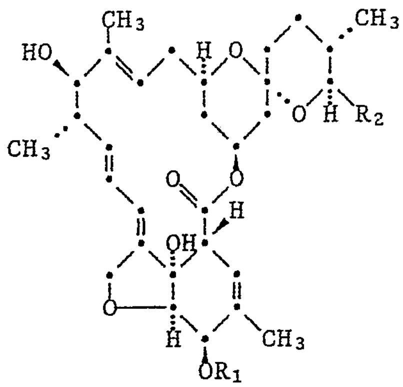

(II),

worin $R_1$ eine Schutzgruppe bedeutet und $R_2$ die unter Formel I angegebene Bedeutung besitzt, mit einem zur Einführung einer 13β-Carbamoyloxygruppe $R_3NH$-C(O)O- geeigneten Reagenz behandelt und danach, sofern ein Produkt mit einer freien 5-Hydroxy-Gruppe erwünscht ist, die Schutzgruppe $R_1$ hydrolytisch abgespaltet.

Als Reagenzien zur Einführung der 13β-Carbamoyloxy Gruppe $R_3NHC(O)O$-eignen sich insbesondere Isocyanate der Formel $R_3$-NCO (III).

Als geeignete Verbindungen der Formel III seien im Rahmen vorliegender Erfindung insbesondere folgende genannt:
Methylisocyanat, tert.-Butylisocyanat, Phenylisocyanat, Chloraethylisocyanat, Allylisocyanat, Benzylisocyanat, 3-Bromphenylisocyanat, Cyclohexylisocyanat, Cyclohexenylisocyanat, 2-Methoxyphenylisocyanat und 4-Nitrophenylisocyanat.

Die Umsetzung erfolgt vorzugsweise in einem inerten, hydroxylgruppenfreien Lösungsmittel in Anwesenheit einer organischen Base, wie z.B. Pyridin, 4-Dimethylaminopyridin, Lutidin, Collidin, Trialkylamin, N-Dialkylanilin, oder bicyclische, nicht nucleophile Basen wie z.B. 1,4-Diazabicyclo[2.2.2]octan (DABCO), 1,5-Diazabicyclo[4.3.0]non-5-en (DBN) oder 1,8-Diazabicyclo[5.4.0]undec-7-en (1,5-5) (DBU). Die Reaktion wird im allgemeinen bei Temperaturen von -10° C bis + 150° C, vorzugsweise von 10° bis 120° C durchgeführt. Man arbeitet dabei zweckmässigerweise in Gegenwart eines reaktionsinerten Lösungsmittels oder Lösungsmittelgemisches. Es eignen sich hierfür z.B. aliphatische und aromatische Kohlenwasserstoffe wie Benzol, Toluol, Xylole, Petrolether, Hexan; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; Ester wie Ethylacetat (Essigsäureethylester), Propylacetat oder Butylacetat; Ketone wie Aceton, Diethylketon, Methylethylketon; Verbindungen wie Dimethylsulfoxid (DMSO), Dimethylformamid (DMF) und Gemische solcher Lösungsmittel untereinander. Man kann die Reaktion aber auch im Ueberschuss einer der oben genannten Basen durchführen.

B. In den Fällen, in denen eine Verbindung der Formel I hergestellt werden soll, worin $R_4$ eine der unter $R_3$ angegebenen Bedeutungen hat, wird ein Allylalkohol der Formel II, worin $R_1$ eine Schutzgruppe bedeutet und $R_2$ die unter Formel I angegebene Bedeutung besitzt, mit einem zur Einführung einer 13β-Carbamoyloxygruppe

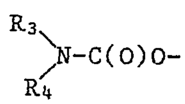

($R_4 \neq H$) geeigneten Reagenz behandelt und danach, sofern ein Produkt mit einer freien 5-Hydroxy-Gruppe erwünscht ist, die Schutzgruppe $R_1$ hydrolytisch abgespaltet.

Als Reagenzien zur Einführung der 13β-Carbamoyloxy-Gruppe

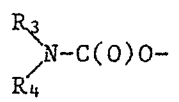

($R_4 \neq H$) eignen sich insbesondere Carbaminsäurehalogenide der Formel IV

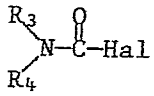 (IV),

($R_4 \neq H$)

worin $R_3$ und $R_4$ unabhängig voneinander eine der unter $R_3$ angegebenen Bedeutungen haben und Hal für Halogen, vorzugsweise für Chlor oder Brom steht.

Als Beispiele geeigneter Verbindungen der Formel IV seien im Rahmen vorliegender Erfindung folgende Vertreter genannt:
Dimethylcarbaminsäurechlorid, Diaethylcarbaminsäurechlorid, Diisopropylcarbaminsäurechlorid, Diphenylcarbaminsäurechlorid und Di-sek.-butylcarbaminsäurechlorid.

Die Reaktion wird bevorzugt in einem der unter Variante A genannten, reaktionsinerten Lösungsmittel und bei Temperaturen von +20°C bis +150°C, vorzugsweise +50°C bis +120°C durchgeführt. Man arbeitet bevorzugt in Gegenwart einer der obengenannten Base.

C. Die Verbindungen der Formel I können auch durch Umsetzung einer Verbindung der Formel V,

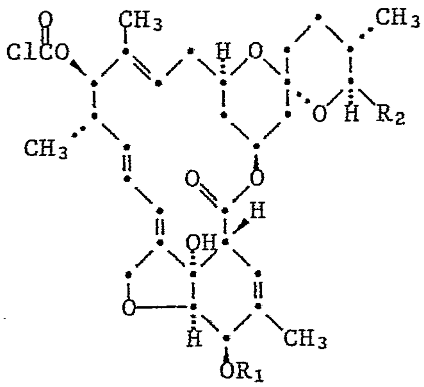 (V),

worin $R_1$ für eine Schutzgruppe steht und $R_2$ eine der unter Formel I angegebenen Bedeutungen hat, mit einem primären oder sekundären Amin der Formel VI,

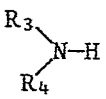 (VI),

worin $R_3$ und $R_4$ wie unter Formel I definiert sind, erhalten werden und sofern ein Produkt mit einer freien 5-Hydroxy-Gruppe erwünscht ist, durch anschliessende hydrolytische Abspaltung der Schutzgruppe $R_1$.

Die Reaktion wird vorzugsweise in einem der unter Variante A genannten Lösungsmittel und bevorzugt in Gegenwart einer der dort genannten Basen bei Temperaturen von -50° bis +50°C, vorzugsweise -20°C bis +30°C durchgeführt.

Die Verbindungen der Formel V lassen sich aus den Verbindungen der Formel II durch Behandlung mit Phosgen ($COCl_2$) herstellen, wobei man vorteilhafterweise bei den unter Variante C angegebenen Bedingungen arbeitet.

In den vorstehend angegebenen Formeln III, IV und VI besitzen $R_3$ und $R_4$ die unter Formel I angegebenen Bedeutungen.

Die Reaktionen zur Herstellung von Verbindungen der Formel I werden vorzugsweise mit Verbindungen der Formeln II oder V durchgeführt, deren reaktive 5-Hydroxi-Gruppe geschützt ist.

Unter OH-Schutzgruppen für den Substituenten $R_1$ sollen hier und im folgenden die in der organische Chemie üblichen und eingangs bereits genannten Schutzfunktionen verstanden werden.

Verbindungen der Formel I, worin $R_1$ eine Schutzgruppe darstellt, lassen sich durch einfache, z.B. hydrolytische Abspaltung der Schutzfunktion in die hochaktiven freien 5-Hydroxi-derivate ($R_1 = H$) überführen und haben somit Zwischenprodukte-Charakter. Im übrigen wird der biologische Wert dieser Verbindungen durch die Schutzgruppe nicht gemindert.

Die Verbindungen der Formel II (13β-Hydroxy-$\Delta^{14,15}$-milbemycine) lassen sich aus Verbindungen der Formel VIII (15-Hydroxy-$\Delta^{13,14}$-milbemycine)

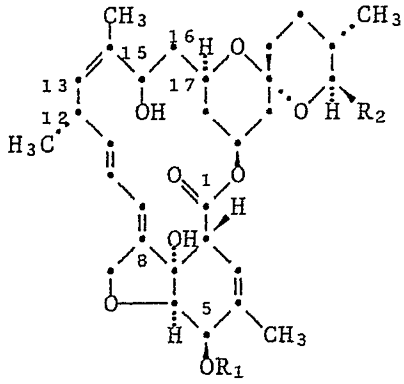

(VIII)

worin $R_2$ eine der unter Formel I angegebenen Bedeutungen hat und $R_1$ für eine Schutzgruppe steht, durch Reaktion mit Pyridiniumdichromat [= $(Pyr)_2+Cr_2O_7$] herstellen. Man arbeitet hierbei in Dimethylformamid und bei Temperaturen zwischen ca. -10° und +60°C. Die $R_1$-Schutzgruppe wird, sofern gewünscht, anschliessend hydrolytisch abgespalten.

Die Verbindungen der Formel VIII [= $\Delta^{13,14}$-15-Hydroxi] lassen sich aus 14,15-Epoxi-Milbemycinen der Formel IX gewinnen, worin $R_1$ und $R_2$ die für die Formel I genannten Bedeutungen haben,

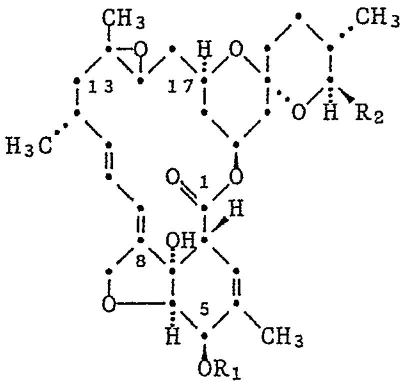

(IX)

mit Hilfe des Komplex-Reagenzes $[HN_3]_m/Al(Ethyl)_3]_n$, worin m und n unabhängig vonienander die Zahl 1 oder 2 oder einen Zahlenwert zwischen 1 und 2 darstellen, in inerten trockenen Lösungsmitteln im Temperaturbereich von -30° bis +100°C, vorzugsweise 0° bis +70°C.

Als inerte Lösungsmittel kommen vorzugsweise aliphatische und aromatische Kohlenwasserstoffe wie Benzol, Toluol, Xylol, Petrolether; Ether wie Diethylether, tert.Butylmethylether, Tetrahydrofuran, Dioxan, Anisol in Frage.

Die Reaktion wird vorteilhaft unter Schutzgas, wie Stickstoff oder Argon, durchgeführt.

Stickstoffwasserstoffsäure $HN_3$ lässt sich in statu nascendi in den $[HN_3]_m/[Al(Et)_3]_n$-Komplex überfuhren, indem man im vorgesehen trockenen Lösungsmittel oder Lösungsmittel-Gemisch Na-Azid suspendiert und daraus mit einer stärkeren Säure, z.B. $H_2SO_4$ (bevorzugt Oleum, um absolut trockene Reaktionsbedingungen zu gewährleisten), $HN_3$ in der Lösung in Freiheit setzt. $Al(Et)_3$ sollte in der Lösung bereits vorliegen oder kurz danach zugegeben werden. Die zur Reaktion vorgesehene Epoxi-Verbindung kann gleichfalls bereits vorliegen oder zu einem geeigneten Zeitpunkt zur Lösung dosiert werden.

Die zur Herstellung der Verbindungen VIII verwendeten Ausgangsverbindungen der Formel IX lassen sich leicht herstellen durch Epoxidierung der aus der US-PS 3,950,360 bekanntgewordenen und ursprünglich als "Antibiotika B-41-A", später als "Milbemycin-A"-Verbindungen und der aus der US-PS 4,346,171 bekanntgewordenen und als "B-41-D" oder "Milbemycin-D" bezeichneten Verbindungen sowie der aus der US-PS 4,173,571 bekanntgewordenen 13-Deoxi-22,23-dihydro-Avermectine ($R_2$ = sec.Butyl) der nachstehenden und bereits eingangs beschriebenen Formel X

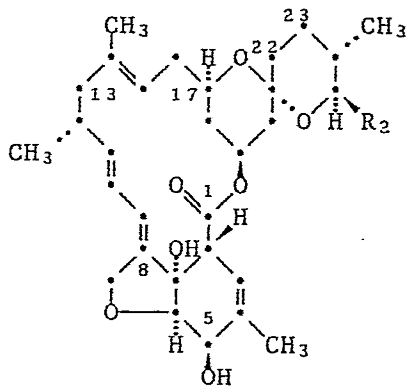

(X)

$R_2 = CH_3$ Milbemycin $A_3$
$R_2 = C_2H_5$ Milbemycin $A_4$
$R_2 = isoC_3H_7$ Milbemycin D
$R_2 = sec.C_4H_9$ 13-Deoxi-22,23-dihydro-C-076-Bla-aglycon.

Die Epoxidierung wird in einer Lösungsmittelphase im Temperaturbereich von -10°C bis +20°C, vorzugsweise -5°C bis +5°C, durchgeführt.

Die Epoxidierung wird mit Persäuren wie Peressigsäure, Trifluorperessigsäure, Perbenzoesäure, Chlorperbenzoesäure durchgeführt.

Durch Acylierung oder Silylierung der 5-OH-Gruppe werden alle jene Derivate der Formeln I, II, V, VIII und IX hergestellt, bei denen $R_1$ eine andere Bedeutung als Wasserstoff ($R_1$ = OH-Schutzgruppe) hat. Die Einführung der Acylgruppe erfolgt üblicherweise mit den entsprechenden Acylhalogeniden oder Acylanhydriden und wird vorzugsweise zur Einführung der eingangs definierten $R_5C(O)$- Gruppe benutzt. Zur Silylierung verwendet man zweckmässigerweise ein Silan der Formel $Y\text{-}Si(R_6)(R_7)(R_8)$, worin $R_6$, $R_7$ und $R_8$ einen der eingangs genannten Reste darstellen, wobei der Begriff Acylhalogenid für Acylchlorid oder Acylbromid steht und wobei Y eine Silylabgangsgruppe bedeutet. Zu den Silylabgangsgruppen Y zählen beispielsweise Bromid, Chlorid, Cyanid, Azid, Acetamid, Trifluoracetat, Trifluormethansulfonat. Diese Aufzählung stellt keine Limitierung dar, der Fachmann kennt weitere typische Silylabgangsgruppen.

5-O-Acylierungen und 5-O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0° bis +80°C, bevorzugt bei +10° bis +40°C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]-undec-7-en (DBU) in Frage.

Die Entfernung dieser Silyl- und Acylreste $R_1$ in der 5-Position geschieht durch selektive milde Hydrolyse ($\rightarrow R_1=H$) mit z.B. Arylsulfonsäure in alkoholischer Lösung oder nach einer anderen dem Fachmann geläufigen Methode.

Das beschriebene Verfahren zur Herstellung der Verbindungen der Formel I ist in allen Teilschritten ein Bestandteil vorliegender Erfindung.

Die Verbindungen der Formel I eignen sich ausgezeichnet zur Bekämpfung von Schädlingen an Tieren und Pflanzen, darunter tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Coccidae, Diaspididae und Eriophydidae (z.B. die Rostmilbe auf Citrusfrüchten): Der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizide und Bodennematizide gegen Schädlinge im Erdboden sowie im Wurzelbereich von Pflanzen geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen,

Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rizoglyphus und andere.

Besonders aber sind die Verbindungen gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meer schweinchen, Ziervögeln. Typische Nematoden dieser Indikation sind: Haemonchus, Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Arten Nematodirus, Cooperia und Oesophagostomum greifen den Intestinaltrakt des Wirtstiers an, während andere der Arten Haemonchus und Ostertagia im Magen und solche der Art Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Sie sind ferner zur Bekämpfung von humanpathogenen Parasiten geeignet, unter denen als typische, im Verdauungstrakt vorkommende Vertreter solche der Arten Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Arten Wuchereria, Brugia, Onchocerca und Loa aus der Familie der Filariidae, die im Blut, im Gewebe und verschiedenen Organen vorkommen, ferner gegen Dracunculus und Parasiten der Arten Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie. z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsufoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie. z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazol-derivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten

Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Napthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgender Publikation beschrieben: "Mc Cutcheon's Detergents and Emulsifiers Annual"
MC Publishing Corp., Ridgewood, New Jersey, 1982.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als mindestens einen Wirkstoff eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### Herstellung von Ausgangs- und Zwischenprodukten

Beispiel A1: Herstellung von 14,15-Epoxi-Milbemycin D (Formel IX)

Zu einer Lösung von 550 mg Milbemycin D in 5 ml Dichlormethan wird unter Eiskühlung eine Lösung von 170 mg Chlorperbenzoesäure in 5 ml Dichlormethan gegeben. Nach 1-stündigem Rühren bei 0° bis +5° C werden nochmals 170 mg des Oxidationsmittels hinzugefügt und weitere 30 min gerührt. Nach beendeter Reaktion wird die Lösung in eine eisgekühlte Lösung von Natriumsulfit gegossen und mit Essigsäureethylester extrahiert. Die vereinigten Extrakte werden einmal mit Wasser gewaschen, getrocknet und im Vakuum eingedampft. Das Rohprodukt wird durch Chromatographierung über eine Silicagel-Säule (Elutionsmittel n-Hexan/Essigsäureethylester 20:15) gereinigt. Es werden 450 mg 14,15-Epoxi-Milbemycin D als amorphe, weisse Substanz erhalten.

Beispiel A2: Herstellung von 15-Hydroxi-$\Delta^{13,14}$-Milbemycin D (Formel VIII)

Es werden bei -20°C zu einer Lösung von 2,1 ml (1,75 g, 15,3 mmol) Triethylaluminium in 8,5 ml abs. Diethylether 9,5 ml (0,41 g, 9,53 mmol) einer 6,69 %igen Lösung von $HN_3$ in Diethylether gegeben, die dann bei -10° C unter stark exothermer Reaktion zu 1,8 g (3,15 mmol) 14,15-Epoxi-Milbemycin D (in Substanz) gegeben wird. Nach 1 Stunde bei Raumtemperatur werden 4 ml absoluter Ether zugegeben und das gallertartige Reaktionsgemisch wird kräftig gerührt. Nach 4 Stunden wird wie in Vorschrift A1 aufgearbeitet und die Chromatographie an 70 g Kieselgel ($CH_2Cl_2$/Aceton 10:1) ergibt 200 mg (10 %) 14-Azido-15-hydroxi-Milbemycin D und 820 g (45 %) 15-Hydroxy-$\Delta^{13,14}$-Milbemycin D, Smp.: 151-153°C (aus Methanol).

Beispiel A3: Herstellung von 5-O-t-Butyldimethylsilyl-14,15-epoxi-Milbemycin D (Formel IX)

Eine Lösung von 2,21 g (3,86 mmol) 14,15-Epoxi-Milbemycin D, 757 mg (5,02 mmol) t-Butyl-dimethylchlorsilan und 342 mg (5,02 mmol) Imidazol in 4 ml DMF wird 90 min bei Raumtemperatur gerührt. Anschliessend werden 80 ml Diethylether zugegeben, und das Gemisch wird über 20 g Kieselgel filtriert und eingeengt. Es werden 2,65 g (100 %) 5-O-t-Butyldimethylsilyl-14,15-epoxi-Milbemycin D erhalten. $^1$H-NMR (300 MHz. Lösungsmittel $CDCl_3$. Messwerte $\delta$ bezogen auf $Si(CH_3)_4$ = TMS).
0,12 ppm (s) $(CH_3)_2Si$-O-;
0,92 ppm (s) (t.-$C_4H_9$)Si-O-;
1,23 ppm (breites s) $C_{14}CH_3$, d.h. Signal der $CH_3$-Gruppe in 14-Position);
2,56 ppm (d; J = 9) $C_{15}H$, d.h. Signal des Protons in 15-Position).

Aehnlich lässt sich durch Reaktion mit Trimethylsilyl-trifluor-methansulfonat das entsprechende 5-O-Trimethylsilyl-14,15-epoxi-Milbemycin D herstellen, Smp. 92-97°C.

Beispiel A4: Herstellung von 5-O-t-Butyldimethylsilyl-15-hydroxi-$\Delta^{13,14}$-Milbemycin D (Formel VIII)

Eine Lösung des $HN_3/Et_3Al$-Komplex-Reagenz (hergestellt aus einer Lösung von 4,97 ml Triethylaluminium in 7 ml abs. Tetrahydrofuran (THF) und 9,15 ml einer 2,39 M Lösung von $HN_3$ (21,9 mmol) in abs. Diethylether) wird unter Argon zu einer Lösung von 5,0 g (7,29 mmol) 5-O-Butyldimethylsilyl-14,15-epoxi-Milbemycin D in ca. 20 ml abs. THF gegeben, und das Gemisch wird 15 Stunden unter Rückfluss erhitzt. Anschliessend werden bei Raumtemperatur 250 ml Ether, 2 ml Methanol und schliesslich ein Gemisch von 10 g $Na_2SO_4 \cdot 10\ H_2O$ und 10 g Celite zugegeben. Das Gemisch wird filtriert, eingeengt, und die Chromatographie des Rohproduktes an 160 g Kieselgel (0 bis 30 % Essigsäureethylester in Hexan) ergibt 2,37 g (47 %) 5-O-t-Butyldimethylsilyl-15-hydroxi-$\Delta^{13,14}$-Milbemycin D.

$^1$H-NMR (300 MHz, $CDCl_3$):

1,59 ppm(d; J = 1), ($C_{14}CH_3$);
4,06 ppm (dd; $J_1$ = 11; $J_2$ = 4) ($C_{15}H$);
5,15 ppm (d; J = 8) ($C_{13}H$):
Daneben werden 109 mg (2 %) 13β-Azido-5-O-6-butyldimethylsilyl-Milbemycin D gewonnen.

Beispiel A5: Herstellung von 14,15-Epoxi-Milbemycin $A_4$ ($R_2 = C_2H_5$)
Zu einer Lösung von 5,7 g (10,5 mmol) Milbemycin $A_4$ in 140 ml Dichlormethan und 120 ml 0,5 M $NaHCO_3$-Lösung wird bei Raumtemperatur eine Lösung von 2,43 g (14,08 mmol) m-Chlorobenzopersäure in 70 ml Dichlormethan tropfenweise zugegeben. Das Gemisch wird 1 Stunde bei Raumtemperatur kräftig gerührt und dann mit 300 ml Dichlormethan verdünnt. Die organische Phase wird mit wässriger $NaHCO_3$-Lösung gewaschen, mit $Na_2SO_4$ getrocknet und eingeengt. Es werden 5,7 g Epoxid als Rohprodukt erhalten.

Beispiel A6: Herstellung von 5-O-t-Butyldimethylsilyl-14,15-epoxi-Milbemycin $A_4$ (Formel IX)
5,7 g 14,15-Epoxi-Milbemycin $A_4$ werden in 10 ml trockenem Dimethylformamid (DMF) gelöst. Bei Raumtemperatur werden 0,63 g (9,16 mmol) Imidazol und 1,4 g (9,34 mmol) t-Butyldimethylchlorsilan zugegeben. Das Gemisch wird 1 Stunde bei Raumtemperatur gerührt und an 150 g Kieselgel chromatographiert (Hexan/Ether 4:1), wobei 2,84 g (40 % d.Th. Ausbeute, bezogen auf Milbemycin $A_4$) des silylierten Epoxi-Derivats erhalten werden.

Beispiel A7: Herstellung von 5-O-t-Butyldimethylsilyl-15-hydroxi-$\Delta^{13,14}$-Milbemycin $A_4$ (Formel VIII)
Das Komplex-Reagenz $HN_3$/Al $(Ethyl)_3$ wird wie folgt hergestellt: 2,8 ml (12,2 mmol) $Al(C_2H_5)_3$ in 4 ml abs. THF werden unter Argon bei ca. -20°C langsam mit 5,28 ml (20,4 mmol) einer 10 %igen Lösung von $HN_3$ in abs. Diethylether versetzt. Zu dieser Lösung wird unter Argon eine Lösung von 2,84 g (4,25 mmol) der im Beispiel A6 erhaltenen Verbindung gegeben, und das so erhaltene Gemisch wird 4 Stunden unter Rückfluss erhitzt. Bei Raumtemperatur werden 500 ml Diethylether, 10 g $Na_2SO_4 \bullet 10H_2O$ und 10 g Celite zugegeben, und das Gemisch wird filtriert und eingeengt. Die Chromatographie des Rohproduktes an 100 g Kieselgel (Hexan/Diethylether 7:2) ergibt 1,72 g (60 % d.Th.) der Titel-Verbindung.
$^1$H-NMR (300 MHz, $CDCl_3$):
1,59 ppm (br. s) ($C_{14}CH_3$);
4,05 ppm (br. s) ($C_{15}H$);
5.15 ppm (d; J = 6) ($C_{13}H$).
Daneben werden 0,1 g 13β-Azido-5-O-t-butyldimethylsilyl-Milbemycin $A_4$ erhalten.

Beispiel A8: Herstellung von 15-Hydroxi-$\Delta^{13,14}$-Milbemycin $A_4$ (Formel VIII)
Die Hydrolyse der im Beispiel A7 genannten Titelverbindung mit einer 1 %igen Lösung von p-Toluolsulfonsäure in Methanol und die Aufarbeitung in Diethylether mit 5 %iger Na-Hydrogencaronat-Lösung ergibt die Titel-Verbindung.

Beispiel A9: Herstellung von 14,15-Epoxi-Milbemycin $A_3$($R_2 = CH_3$) (Formel IX)
Nach der Vorschrift des Beispiels A1 werden aus 220 mg Milbemycin $A_3$ in 5 ml Dichlormethan und 320 mg Benzoepersäure in 5 ml Dichlormethan bei -2°C bis +5°C während 1,5 Stunden und Reinigung über eine Silicagel-Säule 190 mg 14,15-Epoxi-Milbemycin $A_3$ gewonnen.

Beispiel A10: Herstellung von 5-O-tert.-Butyldimethylsilyl-14,15-epoxi-Milbemycin $A_3$ (Formel IX)
Nach der Vorschrift des Beispiels A3 werden aus 190 mg 14,15-Epoxi-Milbemycin $A_3$ und 120 mg tert.-Butyldimethylchlorsilan in Gegenwart von Imidazol 217 mg der Titel-Verbindung erhalten.

Beispiel A11: Herstellung von 5-O-tert.-Butyldimethylsilyl-15-hydroxi-$\Delta^{13,14}$-Milbemycin $A_3$ (Formel VIII)
Analog zur Epoxid-Spaltung des Beispiels A4 werden aus 210 mg 5-O-tert.-Butyldimethylsilyl-14,15-epoxi-Milbemycin $A_3$ in absolutem Diethylether mit Hilfe des Komplex-Reagenz $HN_3/Et_3Al$ unter Argon nach anschliessender Reinigung 203 mg der Titelverbindung erhalten.
$^1$H-NMR (300 MHz, $CDCl_3$):
1,58 ppm (br. s) ($C_{14}CH_3$);
4,05 ppm (br. s) ($C_{15}H$);
5,15 ppm (d; J=6) ($C_{13}H$).

Beispiel A12: Herstellung von 15-Hydroxi-$\Delta^{13,14}$-Milbemycin $A_3$ (Formel VIII)
Analog zu Beispiel A4 wird das Reagenz $HN_3/Al(C_2H_5)_3$ frisch hergestellt und bei -10°C zu einer Lösung von 830 mg (3,05 mmol) 14,15-Epoxi-Milbemycin $A_3$ in 7 ml trockenem Diethylether getropft. Nach der Aufarbeitung werden 385 mg 15-Hydroxi-$\Delta^{13,14}$-Milbemycin $A_3$ und 92 mg 14-Azido-15-hydroxi-Milbemycin $A_3$ erhalten.

Beispiel A13: Herstellung von 13-Deoxi-14,15-epoxi-22,23-dihydro-avermectin-Bla-aglykon ($R_2$ = sec.$C_4H_9$) (Formel IX)

Analog zu Beispiel Al erhält man aus 520 mg 13-Deoxi-22,23-dihydro-avermectin-Bla-aglykon [Tetrahedron Letters, Vol. 24, No. 148, pp. 5333-5336 (1983)] und 210 mg m-Chlorbenzopersäure in 20 ml Dichlormethan 510 mg der Titelverbindung.

Beispiel A14: Herstellung von 5-O-t.Butyldimethylsilyl-13-deoxi-14,15-epoxi-22,23-dihydro-avermectin-Bla-aglykon (Formel IX)

Analog zu Beispiel A3 erhält man aus 220 mg der Titelverbindung von Beispiel A14 und 55 mg tert.Butyldimethylchlorsilan in Gegenwart von 25 mg Imidazol in 5 ml trockenem DMF 108 mg der Titelverbindung.

Beispiel A15: Herstellung von 13-Deoxi-15-hydroxi-$\Delta^{13,14}$-22,23-dihydro-avermectin-Bla-aglykon (Formel VIII)

Analog zu Beispiel A2 erhält man aus 220 mg der Titelverbindung von Beispiel A14 mit dem Komplex-Reagenz, bestehend aus 320 mg $Al(C_2H_5)_3$ und 110 mg einer 6,96 %igen Lösung von $HN_3$ in total 16 ml trockenem Diethylether 112 mg der Titelverbindung. Daneben werden 61 mg 13-Deoxi-14-azido-15-hydroxi-22,23-dihydro-avermectin-Bla-aglykon erhalten.

Beispiel A16: Herstellung von 5-O-tert.Butyldimethylsilyl-13β-hydroxi-milbemycin D und von 13β-Hydroxi-milbemycin D (Formel II)

Eine Lösung bestehend aus 286 mg (0,41 mmol) 5-O-tert.Butyldimethylsilyl-15-hydroxi-$\Delta^{13,14}$-milbemycin D und 209 mg (0,56 mmol) Pyridiniumdichromat (PDC) in 3 ml Dimethylformamid (DMF) wird 30 min bei Raumtemperatur gerührt. Anschliessend wird 1 ml Isopropanol zugegeben, 5 min weitergerührt und dann mit 50 ml Ether verdünnt. Nach weiteren 10 min wird das Gemisch durch Kieselgel filtriert und eingeengt. Bei der Chromatographie des Rohproduktes an 20 g Kieselgel (Ether/Hexan 1:2) werden 165 mg (57 %) 5-O-t-Butyldimethylsilyl-13β-hydroxi-Milbemycin D erhalten.

$^1$H-NMR (300 MHz, $CDCl_3$; TMS):
1,59 ppm (br. s)($C_{14}CH_3$)
3,70 ppm (d; J = 10) ($C_{13}H$).

105 mg (0,153 mmol) der so gewonnenen Verbindung werden mit 1 ml einer 1 %igen Lösung von p-Toluolsulfonsäure in Methanol 1 Stunde bei Raumtemperatur gerührt. Das Gemisch wird mit 20 ml Ether verdünnt, durch Kieselgel filtriert, eingeengt, und der Rückstand wird an ca. 10 g Kieselgel chromatographiert (Aceton/Dichlormethan 1:4), wobei 73 mg (83 %) 13β-Hydroxi-Milbemycin D erhalten werden.

$^1$H-NMR (300 MH; $CDCl_3$; TMS):
1,58 ppm (br. s)($C_{14}CH_3$)
3,71 ppm (d; J = 10)($C_{13}H$).

Herstellung von Endprodukten der Formel I

Beispiel E1: Herstellung von 5-O-tert.-Butyldimethylsilyl-13β-N-methyl-carbamoyloxy-milbemycin $A_4$

Eine Lösung von 75 mg 5-O-tert.-Butyldimethylsilyl-13β-hydroxy-milbemycin $A_4$ und 0,50 ml Methylisocyanat in 1,0 ml Pyridin wird 2 Tage bei Raumtemperatur gerührt. Nach der Aufarbeitung und Chromatographie an Kieselgel mit Hexan/Diaethyläther 1:1 können 64 mg Produkt isoliert werden.

Massenspektrum (MS) (m/e): 729 (M+)

$^1$H-NMR (300 MHz, $CDCl_3$):
2,77 ppm (d, J = 6) ($CH_3NH$)
3,02 ppm (dt, $J_d$ = 3 $J_t$ = 9) ($C_{25}H$)
4,73 ppm (d, J = 11) ($C_{13}H$)

Beispiel E2: Herstellung von 13β-N-Methyl-carbamoyloxy-milbemycin $A_4$

Eine Lösung von 61 mg 5-O-tert.-Butyldimethylsilyl-13β-N-methyl-carbamoxyloxy-milbemycin $A_4$ in 2,0 ml 40 % HF/$CH_3CN$ 5:95 wird 1 Std. bei Raumtemperatur gerührt. Aufarbeitung und Chromatographie an Kieselgel mit Hexan/Essigsäureäthylester ergeben 48 mg Produkt.

Massenspektrum (MS) (m/e): 615 (M+)

$^1$H-NMR (300 MHz, $CDCl_3$):
2,78 ppm (d, J = 6) ($CH_3NH$)
3,03 ppm (dt, $J_d$ = 3, $J_t$ = 10) ($C_{25}H$)
4,83 ppm (d, J = 11) ($C_{13}H$).

Beispiel E3: Herstellung von 5-O-tert.-Butyldimethylsilyl-13β-N-tert.-butyl-carbamoyloxy-milbemycin $A_4$

Eine Lösung von 100 mg 5-O-tert.-Butyldimethylsilyl-13β-hydroxymilbemycin $A_4$ und 0,50 ml tert.-Butylisocyanat in 1,0 ml Pyridin wird 3 Tage auf 100°C erhitzt. Aufarbeitung und Chromatographie an Kieselgel mit Hexan/Essigsäureäthylester 4:1 ergeben 45 mg Produkt.

MS (m/e): 771 (M+)

$^1$H-NMR (300 MHz, $CDCl_3$):

1,30 ppm (s) (($(CH_3)_3C$)
4,80 ppm (bd, J = 11) ($C_{13}H$)

Beispiel E4: Herstellung von 13β-N-tert.-Butyl-carbamoyloxy-milbemycin $A_4$

Eine Lösung von 73 mg 5-O-tert.-Butyldimethyl-13β-N-tert.-butyl-carbamoyloxy-milbemycin $A_4$ in 1,0 ml Methylenchlorid wird mit 1,0 ml 40 % HF/$CH_3CN$ 5:95 versetzt, und die erhaltene Reaktionslösung lässt man 1 Std. bei Raumtemperatur rühren. Aufarbeitung und Chromatographie an Kieselgel mit Hexan/Essigsäureäthylester 2:1 ergeben 42 mg Produkt.

MS (m/e): 657 (M+)
$^1$H-NMR (300 MHz, $CDCl_3$):
1,30 ppm (s) (($CH_3)_3C$)
3,02 ppm (dt, $J_d$ = 3, $J_t$ = 10) ($C_{25}H$)
4,80 ppm (bd, J = 10) ($C_{13}H$)

Beispiel E5: Herstellung von 5-O-tert.-Butyldimethylsilyl-13β-N-phenylcarbamoyloxy-milbemycin $A_4$

Eine Lösung von 75 mg 5-tert.-Butyldimethylsilyl-13β-hydroxy-milbemycin $A_4$ und 0,50 ml Phenylisocyanat in 1,0 ml Pyridin wird 3 Std. bei Raumtempteratur gerührt. Aufarbeitung und Chromatographie an Kieselgel mit Hexan/Essigsäureäthylester ergeben 63 mg Produkt.

MS (m/e): 791 (M+)
$^1$H-NMR (300 MHz, $CDCl_3$):
3,02 ppm (dt, $J_d$ = 3, $J_t$ = 10)
4,92 ppm (d, J = 11) ($C_{13}H$)
7,04 ppm (bt, J = 8) NH-$C_6H_4$-$\underline{H}$(4))

Beispiel E6: Herstellung von 13β-N-Phenyl-carbamoyloxy-milbemycin $A_4$

Eine Lösung von 57 mg 5-tert.-Butyldimethylsilyl-13β-N-phenyl-milbemycin $A_4$ in 1,0 ml 40 % HF/$CH_3CN$ 5:95 wird eine Stunde bei Raumtemperatur gerührt. Aufarbeitung und Chromatographie an Kieselgel mit Methylenchlorid/Methanol (2 %) ergeben 41 mg Produkt.

MS (m/e): 677 (M+)
$^1$H-NMR (300 MHz, $CDCl_3$):
3,03 ppm (dt, $J_d$ = 3, $J_t$ = 10) ($C_{25}H$)
4,92 ppm (d, J = 11) ($C_{13}H$) 7,05 ppm (bt, J = 8) (NH-$C_6H_4$-$\underline{H}$(4))

Beispiel E7: Herstellung von 5-tert.-Butyldimethylsilyl-13β-N,N-diisopropyl-carbamoyloxy-milbemycin $A_4$

Eine Lösung von 150 mg 5-O-tert.-Butyldimethylsilyl-13β-hydroxy-milbemycin $A_4$ und 171 mg Diisopropylcarbaminsäurechlorid in 1,5 ml Pyridin wird 17 Std. auf 80°C erhitzt. Aufarbeitung und Chromatographie an Kieselgel mit Hexan/Essigsäureäthylester 6:1 ergeben 56 mg Produkt.

$^1$H-NMR (300 MHz, $CDCl_3$):
1,21 ppm (bd, J = 7) (($\underline{CH_3}$)$_2$CH)
3,01 ppm (dt, $J_d$ = 3, $J_t$ = 10) ($C_{25}H$)
4,95 ppm (d, J = 12) ($C_{13}H$)

Beispiel E8: Herstellung von 13β-N,N-Diisopropyl-carbamoyloxy-milbemycin $A_4$

Eine Lösung von 90 mg 5-tert.-Butyldimethylsilyl-13β-N,N-diisopropylcarbamoyloxy-milbemycin $A_4$ in 0,5 ml 40 % HF/$CH_3CN$ 5:95 wird 90 Min. bei Raumtemperatur gerührt. Aufarbeitung und Chromatographie an Kieselgel mit Hexan/Essigsäureäthylester 2:1 ergeben 70 mg Produkt.

$^1$H-NMR (300 MHz, $CDCl_3$):
1,20 ppm (bd, J = 7) (($\underline{CH_3}$)$_2$CH)
3,02 ppm (dt, $J_d$ = 3, $J_t$ = 10) ($C_{25}H$);
4,96 ppm (d, J = 11) ($C_{13}H$)

Nach den vorstehend beschriebenen Verfahren lassen sich beispielsweise auch folgende Verbindungen der Formel I herstellen:

Beispiel E9: 5-O-tert.-Butyldimethylsilyl-13β-N-methyl-carbamoyloxy-milbemycin D

$^1$H-NMR (300 MHz, $CDCl_3$):
2,79 ppm (d, J = 5) ($\underline{CH_3}$NH)
3,03 ppm (bd, J = 10) ($C_{25}H$)
4,84 ppm (d, J = 11) ($C_{13}H$)

Beispiel E10: 13β-N-Methylcarbamoyloxy-milbemycin D

$^1$H-NMR (300 MHz, $CDCl_3$):
2,80 ppm (d, J = 5) ($\underline{CH_3}$NH);
3,04 ppm (bd, J = 9) ($C_{25}H$);
4,84 ppm (d, J = 11) ($C_{13}H$).

Beispiel E11: 5-O-tert.-Butyldimethylsilyl-13β-N-cyclohexylcarbamoyloxy-milbemycin $A_4$
MS (m/e): 797 ($M^+$)
$^1$H-NMR (300 MHz, $CDCl_3$):
3,02 ppm (dt, $J_d = 3$, $J_t = 9$) ($C_{25}H$)
4,82 ppm (bd, J = 11) ($C_{13}H$)

Beispiel E12: 13β-N-Cyclohexylcarbamoyloxy-milbemycin $A_4$
MS (m/e): 683 ($M^+$)
$^1$H-NMR (300 MHz, $CDCl_3$):
3,05 ppm (dt, $J_d = 3$, $J_t = 9$) ($C_{25}H$)
4,82 ppm (bd, J = 11) ($C_{13}H$)

Beispiel E13: 5-O-tert.-Butyldimethylsilyl-13β-N-(4-bromphenyl)carbamoyloxy-milbemycin $A_4$
MS (m/e): 869, 871 ($M^+$)
$^1$H-NMR (300 MHz, $CDCl_3$):
3,02 ppm (bt, J = 9) ($C_{25}H$)
4,90 ppm (d, J = 11) ($C_{13}H$)

Beispiel E14: 13β-N-(4-Bromphenyl)carbamoyloxy-milbemycin $A_4$
MS (m/e): 755, 757 ($M^+$)
$^1$H-NMR (300 MHz, $CDCl_3$):
3,01 ppm (dt, $J_d = 3$, $J_t = 9$) ($C_{25}H$)
4,90 ppm (d, J = 12) ($C_{13}H$)

Beispiel E15: Herstellung von 5-O-tert.-Butyldimethylsilyl-13β-N,N-dimethyl-carbamoyloxy-milbemycin $A_4$
MS (m/e): 743 ($M^+$, $C_{41}H_{65}NO_9Si$)
$^1$H-NMR (300 MHz, $CDCl_3$):
2,90 ppm (6s) ($(CH_3)_2N$)
3,03 ppm (dt, $J_d = 3$, $J_t = 9$) ($C_{25}H$)
4,42 ppm (6s, $W_{1/2} = 10$) ($C_{13}H$)
4,83 ppm (d, J = 11) ($C_{13}H$)

Beispiel E16: Herstellung von 13β-N,N-Dimethyl-carbamoyloyx-milbemycin $A_4$
MS (m/e): 629 ($M^+$, $C_{35}H_{51}NO_9$)
$^1$H-NMR (300 MHz, $CDCl_3$):
2,89 ppm (6s) ($(CH_3)_2N$)
3,03 ppm (dt, $J_d = 2{,}5$, $J_t = 9$) ($C_{25}H$)
4,29 ppm (t, J = 7) ($C_5H$)
4,83 ppm (d, J = 11) ($C_{13}H$)

Beispiel E17: Herstellung von 5-O-tert.-Butyldimethyl-13β-N-ethyl-carbamoyloxy-milbemycin $A_4$
MS (m/e): 743 ($M^+$, $C_{41}H_{65}NO_9Si$)
$^1$H-NMR (300 MHz, $CDCl_3$):
3,03 ppm (dt, $J_d = 2{,}5$, $J_t = 9$) ($C_{25}H$)
3,20 ppm (m) $CH_{25}N$)
4,42 ppm (6s, $W_{1/2} = 11$) ($C_5H$)
4,81 ppm (6d, J = 11) ($C_{13}H$)

Beispiel E18: Herstellung von 13β-N-Ethyl-carbamoyloxy-milbemycin $A_4$
MS (m/e): 629 ($M^+$, $C_{35}H_{51}NO_9$)
$^1$H-NMR (300 MHz, $CDCl_3$):
3,03 ppm (dt, $J_d = 2{,}5$, $J_t = 9$) ($C_{25}H$)
3,20 ppm (m) ($CH_2N$)
4,28 ppm (6d, J = 6) ($C_5H$)
4,83 ppm (6d, J = 11) ($C_{13}H$)

Beispiel E19: Herstellung von 5-O-tert.-Butyldimethylsilyl-13β-N-(4-nitrophenyl)-carbamoyloxy-milbemycin $A_4$
MS (m/e): 836 ($M^+$, $C_{45}H_{64}N_2O_{11}Si$)
$^1$H-NMR (300 MHz, $CDCl_3$):
3,03 ppm (dt, $J_d = 2{,}5$, $J_t = 9$) ($C_{25}H$)
4,42 ppm (6s, $W_{1/2} = 11$) ($C_5H$)
4,93 ppm (d, J = 6) ($C_{13}H$) 7,53 ppm (m) und 8,19 ppm (m) (aromatische Ringprotonen

Beispiel E20: Herstellung von 13β-N-(4-Nitrophenyl)-carbamoyloxy-milbemycin $A_4$
MS (m/e): 722 (M+, $C_{39}H_{50}N_2O_{11}$))
$^1$H-NMR (300 MHz, $CDCl_3$):
3,03 ppm (dt, $J_d$ = 2,5, $J_t$ = 9) ($C_{25}H$)
4,29 ppm (t, J = 7) ($C_5H$)
4,93 ppm (d, J = 11) ($C_{13}H$)
7,53 ppm (m) und 8,20 ppm (m) (aromatische Ringprotonen)

Beispiel E21: Herstellung von 5-O-tert.-Butyldimethylsilyl-13β-N-(2-trifluormethylphenyl)-carbamoyloxy-milbemycin $A_4$
MS (m/e): 859 (M+, $C_{46}H_{64}F_3O_9Si$)
$^1$H-NMR (300 MHz, $CDCl_3$):
3,03 ppm (dt, $J_d$ = 2,5, $J_t$ = 9) ($C_{25}H$)
4,42 ppm (6s, $W_{1/2}$ = 11) ($C_5H$)
4,90 ppm (d, J = 10) ($C_{13}H$)

Beispiel E22: Herstellung von 13β-N-(2-Trifluorphenyl)-carbamoyloxy-milbemycin $A_4$
MS (m/e): 745 (M+, $C_{40}H_{50}F_3NO_9$)
$^1$H-NMR (300 MHz, $CDCl_3$):
3,03 ppm (dt, $J_d$ = 2,5, $J_t$ = 9) ($C_{25}H$)
4,28 ppm (t, J = 7) ($C_5H$)
4,90 ppm (d, J = 10) ($C_{13}H$)

Beispiel E23: Herstellung von 5-O-tert.-Butyldimethylsilyl-13β-N-trichlormethyl-carbamoyloxy-milbemycin $A_4$
$^1$H-NMR (300 MHz, $CDCl_3$):
3,03 ppm (dt, $J_d$ = 2,5, $J_t$ = 9) ($C_{25}H$)
4,42 ppm (6s, $W_{1/2}$ = 10) ($C_5H$)
4,91 ppm (d, J = 11) ($C_{13}H$)

Beispiel E24: Herstellung von 13β-Carbamoyloxy-milbemycin $A_4$
MS (m/e): 601 (M+, $C_{33}H_{47}NO_9$)
$^1$H-NMR (300 MHz, $CDCl_3$):
3,03 ppm (dt, $J_d$ = 2,5, $J_t$ = 9) ($C_{25}H$)
4,28 ppm (6d, J = 6) ($C_5H$)
4,80 ppm (d, J = 11) ($C_{13}H$)

Beispiel E25: Herstellung von 5-O-tert.-Butyldimethylsilyl-13β-N-isopropyl-carbamoyloxy-milbemycin $A_4$
MS (m/e): 757 (M+, $C_{42}H_{67}NO_9Si$)
$^1$H-NMR (300 MHz, $CDCl_3$):
3,03 ppm (dt, $J_d$ = 2,5, $J_t$ = 9) ($C_{25}H$)
3,57 ppm (m) (($CH_3)_2C\underline{H}NH$)
4,42 ppm (6s, $W_{1/2}$ = 10) ($C_5H$)
4,81 ppm (6d, J = 11) ($C_{13}H$)

Beispiel E26: Herstellung von 13β-Isopropyl-carbamoyloxy-milbemycin $A_4$
MS (m/e): 643 (M+, $C_{36}H_{53}NO_9$)
$^1$H-NMR (300 MHz, $CDCl_3$):
3,03 ppm (dt, $J_d$ = 2,5, $J_t$ = 9) ($C_{25}H$)
3,77 ppm (m) (($CH_3)_2C\underline{N}NH$)
4,28 ppm (t, J = 7) ($C_5\underline{H}$)
4,82 ppm (6d, J = 11) ($C_{13}H$)

Beispiel E27: Herstellung von 5-O-tert.-Butyldimethylsilyl-13β-N-benzyl-carbamoyloxy-milbemycin $A_4$
MS (m/e): 805 (M+, $C_{46}H_{67}NO_9Si$)
$^1$H-NMR (300 MHz, $CDCl_3$):
3,03 ppm (dt, $J_d$ = 2,5, $J_t$ = 9) ($C_{25}H$)
4,85 ppm (d, J = 10) ($C_{13}H$)
7,17-7,42 ppm (m) ($C_6H_5$)

Beispiel E28: Herstellung von 13β-N-Benzyl-carbamoyloxy-milbemycin $A_4$
MS (m/e): 691 (M+, $C_{40}H_{53}NO_9$)
$^1$H-NMR (300 MHz, $CDCl_3$):
3,03 ppm (dt, $J_d$ = 2,5, $J_t$ = 9) ($C_{25}H$)
4,87 ppm (d, J = 10) ($C_{13}H$)
7,20-7,37 ppm (m) ($C_6H_5$)

Beispiel E29: Herstellung von 5-O-tert.-Butyldimethylsilyl-13β-N-((S)-1-phenylethyl)-carbamoyloxy-milbemycin $A_4$

MS (m/e): 819 ($M^+$, $C_{47}H_{49}NO_9Si$)

$^1$H-NMR (300 MHz, $CDCl_3$):

3,03 ppm (bt, J = 9) ($C_{25}H$)

4,42 ppm (6s, $W_{1/2}$ = 10) ($C_5H$)

4,67 - 4,95 ppm (m) ($C_{13}\underline{H}$; $C_6H_5$-C$\underline{H}$($CH_3$)N$\underline{H}$-)

7,17 - 7,37 ppm (m) ($C_6\underline{H_5}$)

Beispiel E30: Herstellung von 13β-N-((S)-1-Phenylethyl)-carbamoyloxy-milbemycin $A_4$

MS (m/e): 705 ($M^+$, $C_{41}H_{55}NO_9$)

$^1$H-NMR (300 MHz, $CDCl_3$):

3,03 ppm (dt, $J_d$ = 2,5, $J_t$ = 9) ($C_{25}H$)

4,28 ppm (t, J = 7) ($C_5H$)

4,63 - 4,94 ppm (m) ($C_{13}\underline{H}$; $C_6H_5$-C$\underline{H}$($CH_3$)N$\underline{H}$-)

7,15 - 7,37 ppm (m) ($C_6\underline{H_5}$)

Beispiel E31: Herstellung von 5-O-tert.-Butyldimethylsilyl-13β-N-((R)-1-phenylethyl)-carbamoyloxy-milbemycin $A_4$

MS (m/e): 819 ($M^+$, $C_{47}H_{49}NO_9Si$)

$^1$H-NMR (300 MHz, $CDCl_3$):

3,03 ppm (dt, $J_d$ = 2,5, $J_t$ = 9) ($C_{25}H$)

4,42 ppm (6s, $W_{1/2}$ = 10) ($C_5H$)

4,67 - 5,00 ppm (m) ($C_{13}\underline{H}$; $C_6H_5$-C$\underline{H}$($CH_3$)N$\underline{H}$-)

7,10 - 7,38 ppm (m) ($C_6\underline{H_5}$)

Beispiel E32: Herstellung von 13β-N-((R)-1-Phenylethyl)-carbamoyloxy-milbemycin $A_4$

MS (m/e): 705 ($M^+$, $C_{41}H_{55}NO_9$)

$^1$H-NMR (300 MHz, $CDCl_3$):

3,03 ppm (dt, $J_d$ = 2,5, $J_t$ = 9) ($C_{25}H$)

4,28 ppm (t, J = 7) ($C_5H$)

4,63 - 4,98 ppm (m) ($C_{13}\underline{H}$; $C_6H_5$-C$\underline{H}$($CH_3$)N$\underline{H}$-)

7,13 - 7,37 ppm (m) $C_6\underline{H_5}$)

Beispiel E33: Herstellung von 5-O-tert.-Butyldimethylsilyl-13β-N-(2-bromphenyl)-carbamoyloxy-milbemycin $A_4$

MS (m/e): 871, 869 ($M^+$, $C_{45}H_{64}BrNO_9Si$)

$^1$H-NMR (300 MHz, $CDCl_3$):

3,03 ppm (dt, $J_d$ = 2,5, $J_t$ = 9) ($C_{25}H$)

4,43 ppm (bs, $W_{1/2}$ = 10) ($C_5H$)

4,92 ppm (d, J = 10) ($C_{13}H$)

6,93 ppm (m), 7,27 ppm (m), 7,50 ppm und und 8,14 ppm (m) (aromatische Ringprotonen)

Beispiel 34: Herstellung von 13β-N-(2-Bromphenyl)-carbamoyloxy-milbemycin $A_4$

MS (m/e): 757, 755 ($M^+$, $C_{39}H_{50}BrNO_9$)

$^1$H-NMR (300 MHz, $CDCl_3$):

3,03 ppm (dt, $J_d$ = 2,5, $J_t$ = 9) ($C_{25}H$)

4,28 ppm (t, J = 7) ($C_5H$)

4,92 ppm (d, J = 10) ($C_{13}H$)

6,92 ppm (m), 7,27 ppm (m), 7,50 ppm und und 8,13 ppm (m) (aromatische Ringprotonen)

Beispiel E35: Herstellung von 5-O-tert.-Butyldimethylsilyl-13β-N-(2-ethylphenyl)-carbamoyloxy-milbemycin $A_4$

MS (m/e): 819 ($M^+$, $C_{47}H_{69}NO_9Si$)

$^1$H-NMR (300 MHz, $CDCl_3$):

2,57 ppm (q, J = 8) ($C_6H_4$-C$\underline{H_2}$$CH_3$)

3,03 ppm (dt, $J_d$ = 2,5, $J_t$ = 9) ($C_{25}H$)

4,42 ppm (bs, $W_{1/2}$ = 10) ($C_5H$)

4,92 ppm (d, J = 10) ($C_{13}H$)

Beispiel E36: Herstellung von 13β-N-(2-Ethylphenyl)-carbamoyloxy-milbemycin $A_4$

MS (m/e): 705 ($M^+$, $C_{41}H_{55}NO_9$)

$^1$H-NMR (300 MHz, $CDCl_3$):

2,58 ppm (q, J = 8) ($C_6H_4$-C$\underline{H_2}$$CH_3$)

3,03 ppm (dt, $J_d$ = 2,5, $J_t$ = 9) ($C_{25}H$)

4,28 ppm (t, J = 7) ($C_5H$)
4,92 ppm (d, J = 11) ($C_{13}H$)

Beispiel E37: Herstellung von 5-O-tert.-Butyldimethylsilyl-13β-N-(2-nitrophenyl)-carbamoyloxy-milbemycin $A_4$
MS (m/e): 836 ($M^+$, $C_{45}H_{64}N_2O_{11}Si$)
$^1H$-NMR (300 MHz, $CDCl_3$):
3,03 ppm (dt, $J_d$ = 2,5, $J_t$ = 9) ($C_{25}H$)
4,43 ppm (bs, $W_{1/2}$ = 10) ($C_5H$)
4,92 ppm (d, J = 11) ($C_{13}H$)
7,12 ppm (m), 7,60 ppm (m), 8,12 ppm und und 8,57 ppm (m) (aromatische Ringprotonen)

Beispiel E38: Herstellung von 13β-N-(2-Nitrophenyl-carbamoyloxy-milbemycin $A_4$
MS (m/e): 722 ($M^+$, $C_{39}H_{50}N_2O_{11}$)
$^1H$-NMR (300 MHz, $CDCl_3$):
3,03 ppm (dt, $J_d$ = 2,5, $J_t$ = 9) ($C_{25}H$)
4,29 ppm (t, J = 7) ($C_5H$)
4,93 ppm (d, J = 10) ($C_{13}H$)
7,10 ppm (m), 7,61 ppm (m), 8,12 ppm und und 8,57 ppm (m) (aromatische Ringprotonen)

Beispiel E39: Herstellung von
5-O-tert.-Butyldimethylsilyl-13β-N-(2,6-dimethylphenyl)-carbamoyloxy-milbemycin $A_4$
MS (m/e): 819 ($M^+$, $C_{47}H_{69}NO_9Si$)
$^1H$-NMR (300 MHz, $CDCl_3$):
2,22 ppm (s) ($C_6H_3$-$(CH_3)_2$
3,03 ppm (dt, $J_d$ = 2,5, $J_t$ = 9) ($C_{25}H$)
4,42 ppm (bs, $W_{1/2}$ = 10) ($C_5H$)
4,88 ppm (d, J = 11) ($C_{13}H$)
7,06 ppm (m) ($C_6H_3$)

Beispiel E40: Herstellung von 13β-N-(2,6-Dimethylphenyl)-carbamoyloxy-milbemycin $A_4$
MS (m/e): 705 ($M^+$, $C_{41}H_{55}NO_9$)
$^1H$-NMR (300 MHz, $CDCl_3$):
2,22 ppm (s) $C_6H_3$-$(CH_3)_2$
3,03 ppm (dt, $J_d$ = 2,5, $J_t$ = 9) ($C_{25}H$)
4,28 ppm (bt, J = 7) ($C_5H$)
4,89 ppm (bd, J = 10) ($C_{13}H$)
7,06 ppm (m) ($C_6H_3$)

Beispiel E41: Herstellung von 5-O-tert.-Butyldimethylsilyl-13β-N-(2,6-chlorethyl)-carbamoyloxy-milbemycin $A_4$
MS (m/e): 777 ($M^+$, $C_{41}H_{64}ClNO_9Si$)
$^1H$-NMR (300 MHz, $CDCl_3$):
3,03 ppm (dt, $J_d$ = 2,5, $J_t$ = 9) ($C_{25}H$)
3,43-3,88 ppm (m) ($C_{17}H$; $ClCH_2CH_2NH$)
4,41 ppm (bs, $W_{1/2}$ = 10) ($C_5H$)
4,81 ppm (d, J = 11) ($C_{13}H$)

Beispiel E42: Herstellung von 13β-N-(2-Chlorethyl-carbamoyloxy-milbemycin $A_4$
MS (m/e): 663 ($M^+$, $C_{35}H_{50}ClNO_9$)
$^1H$-NMR (300 MHz, $CDCl_3$):
3,03 ppm (dt, $J_d$ = 2,5, $J_t$ = 9) ($C_{25}H$)
3,40-3,67 ppm (m) ($C_{17}H$; $ClCH_2CH_2NH$)
4,28 ppm (t, J = 7) ($C_5H$)
4,82 ppm (d, J = 11) ($C_{13}H$)

Beispiel E43: Herstellung von 5-O-tert.-Butyldimethylsilyl-13β-N-(1-naphthyl)-carbamoyloxy-milbemycin $A_4$
MS (m/e): 841 ($M^+$, $C_{49}H_{67}NO_9Si$)
$^1H$-NMR (300 MHz, $CDCl_3$):
3,03 ppm (dt, $J_d$ = 2,5, $J_t$ = 9) ($C_{25}H$)
4,42 ppm (bd, $W_{1/2}$ = 10) ($C_5H$)
4,97 ppm (d, J = 10) ($C_{13}H$)

Beispiel E44: Herstellung von 13N-(1-Naphthyl)-carbamoyloxy-milbemycin $A_4$
MS (m/e): 727 ($M^+$, $C_{43}H_{53}NO_9$)
$^1H$-NMR (300 MHz, $CDCl_3$):
3,03 ppm (dt, $J_d$ = 2,5, $J_t$ = 9) ($C_{25}H$)

4,28 ppm (t, J = 7) ($C_5H$)
4,99 ppm (d, J = 10) ($C_{13}H$)

Beispiel E45: Herstellung von 5-O-tert.-Butyldimethylsilyl-13β-N-methyl-carbamoyloxy-milbemycin $A_3$
MS (m/e): 715 ($M^+$, $C_{39}H_{61}O_9Si$)
$^1H$-NMR (300 MHz, $CDCl_3$):
2,76 ppm (d, J = 6) ($C\underline{H}_3NH$)
3,25 ppm (m) ($C_{25}H$)
4,82 ppm (d, J = 11) ($C_{13}H$)

Beispiel E46: Herstellung von 13β-N-Methyl-carbamoyloxy-milbemycin $A_3$
MS (m/e): 601 ($M^+$, $C_{33}H_{47}NO_9$)
$^1H$-NMR (300 MHz, $CDCl_3$):
2,78 ppm (d, J = 6) ($C\underline{H}_3NH$)
3,25 ppm (m) ($C_{25}H$)
4,83 ppm (d, J = 11) ($C_{13}H$)

Analog zu den beschriebenen Arbeitsweisen werden auch die nachfolgend genannten Verbindungen der Formel I hergestellt:

| Verb. Nr. | $R_3$ | $R_4$ | $R_1$ | Physik.Konst. bzw. Beispiel |
|---|---|---|---|---|
| 1.1 | $CH_3$ | H | H | E46 |
| 1.2 | $C_2H_5$ | H | H | |
| 1.3 | $C_3H_7$-n | H | H | |
| 1.4 | $C_3H_7$-iso | H | H | |
| 1.5 | $C_4H_9$-n | H | H | |
| 1.6 | $CH_2CH_2Cl$ | H | H | |
| 1.7 | Cyclohexyl | H | H | |
| 1.8 | $CH_2CH{=}CH_2$ | H | H | |
| 1.9 | 1-Cyclohexan-1-yl | H | H | |
| 1.10 | Benzyl | H | H | |
| 1.11 | $C_6H_4Br(4)$ | H | H | |
| 1.12 | $C_6H_4Cl(3)$ | H | H | |
| 1.13 | $C_6H_4F(2)$ | H | H | |
| 1.14 | $C_6H_3Cl_2(3,4)$ | H | H | |
| 1.15 | $C_6H_3F_2(2,4)$ | H | H | |
| 1.16 | $C_6H_4CH_3(4)$ | H | H | |
| 1.17 | $C_6H_4OC_2H_5(2)$ | H | H | |
| 1.18 | $C_6H_4OCH_3(3)$ | H | H | |
| 1.19 | $C_6H_4NO_2(4)$ | H | H | |
| 1.20 | $C_6H_3F(4)NO_2(3)$ | H | H | |
| 1.21 | $CH_3$ | $CH_3$ | H | |
| 1.22 | $C_2H_5$ | $C_2H_5$ | H | |
| 1.23 | $C_3H_7$-n | $C_3H_7$-n | H | |
| 1.24 | $C_3H_7$-iso | $C_3H_7$-iso | H | |
| 1.25 | $C_4H_9$-tert | $C_4H_9$-tert | H | |
| 1.26 | $C_4H_9$-sek | $C_4H_9$-sek | H | |
| 1.27 | Phenyl | Phenyl | H | |
| 1.28 | $C_6H_{13}$-n | H | H | |
| 1.29 | $CH_3$ | H | $(C_4H_9\text{-t})Si(CH_3)_2$ | E45 |
| 1.30 | $C_2H_5$ | H | $(C_4H_9\text{-t})Si(CH_3)_2$ | |

Fortsetzung von Tabelle 1:

| Verb. Nr. | $R_3$ | $R_4$ | $R_1$ | Physik.Konst. bzw. Beispiel |
|---|---|---|---|---|
| 1.31 | $C_3H_7$-n | H | $(C_4H_9\text{-t})Si(CH_3)_2$ | |
| 1.32 | $C_3H_7$-iso | H | $(C_4H_9\text{-t})Si(CH_3)_2$ | |
| 1.33 | $C_4H_9$-n | H | $(C_4H_9\text{-t})Si(CH_3)_2$ | |
| 1.34 | $C_6H_4F(2)$ | H | $(C_4H_9\text{-t})Si(CH_3)_2$ | |
| 1.35 | $CH_3$ | H | $(C_4H_9\text{-t})Si(C_6H_5)_2$ | |
| 1.36 | $C_2H_5$ | H | $(C_3H_7\text{-iso})_2SiCH_3$ | |
| 1.37 | $C_3H_7$-n | H | $Si(C_6H_5)_3$ | |
| 1.38 | $CH_3$ | $CH_3$ | $(C_4H_9\text{-t})Si(CH_3)_2$ | |

Tabelle 2: Typische Vertreter von Milbemycin-$A_4$-derivaten der Formel I ($R_2$ = $C_2H_5$):

| Verb. Nr. | $R_3$ | $R_4$ | $R_1$ | Physik.Konst. bzw. Beispiel |
|---|---|---|---|---|
| 2.1 | $CH_3$ | H | H | E2 |
| 2.2 | $C_2H_5$ | H | H | E18 |
| 2.3 | $C_3H_7$-n | H | H | |
| 2.4 | $C_3H_7$-iso | H | H | E26 |
| 2.5 | $C_4H_9$-n | H | H | |
| 2.6 | $CH_2CH_2Cl$ | H | H | E42 |
| 2.7 | Cyclohexyl | H | H | |
| 2.8 | $CH_2CH{=}CH_2$ | H | H | |
| 2.9 | 1-Cyclohexan-1-yl | H | H | |
| 2.10 | Benzyl | H | H | E28 |
| 2.11 | $C_6H_4Br(4)$ | H | H | E14 |
| 2.12 | $C_6H_4Cl(3)$ | H | H | |
| 2.13 | $C_6H_4F(2)$ | H | H | |
| 2.14 | $C_6H_3Cl_2(3,4)$ | H | H | |
| 2.15 | $C_6H_3F_2(2,4)$ | H | H | |
| 2.16 | $C_6H_4CH_3(4)$ | H | H | |

Fortsetzung von Tabelle 2:

| Verb. Nr. | $R_3$ | $R_4$ | $R_1$ | Physik.Konst. bzw. Beispiel |
|---|---|---|---|---|
| 2.17 | $C_6H_4OC_2H_5(2)$ | H | H | |
| 2.18 | $C_6H_4OCH_3(3)$ | H | H | |
| 2.19 | $C_6H_4NO_2(4)$ | H | H | E20 |
| 2.20 | $C_6H_3F(4)NO_2(3)$ | H | H | |
| 2.21 | $CH_3$ | $CH_3$ | H | E16 |
| 2.22 | $C_2H_5$ | $C_2H_5$ | H | |
| 2.23 | $C_3H_7$-n | $C_3H_7$-n | H | |
| 2.24 | $C_3H_7$-iso | $C_3H_7$-iso | H | |
| 2.25 | $C_4H_9$-tert | $C_4H_9$-tert | H | |
| 2.26 | $C_4H_9$-sek | $C_4H_9$-sek | H | |
| 2.27 | Phenyl | Phenyl | H | |
| 2.28 | $C_6H_{13}$-n | H | H | |
| 2.29 | $CH_3$ | H | $(C_4H_9\text{-t})Si(CH_3)_2$ | E1 |
| 2.30 | $C_2H_5$ | H | $(C_4H_9\text{-t})Si(CH_3)_2$ | E17 |
| 2.31 | $C_3H_7$-n | H | $(C_4H_9\text{-t})Si(CH_3)_2$ | |
| 2.32 | $C_3H_7$-iso | H | $(C_4H_9\text{-t})Si(CH_3)_2$ | E25 |
| 2.33 | $C_4H_9$-n | H | $(C_4H_9\text{-t})Si(CH_3)_2$ | |
| 2.34 | $C_6H_4F(2)$ | H | $(C_4H_9\text{-t})Si(CH_3)_2$ | |
| 2.35 | $CH_3$ | H | $(C_4H_9\text{-t})Si(C_6H_5)_2$ | |
| 2.36 | $C_2H_5$ | H | $(C_3H_7\text{-iso})_2SiCH_3$ | |
| 2.37 | $C_3H_7$-n | H | $Si(C_6H_5)_3$ | |
| 2.38 | $CH_3$ | $CH_3$ | $(C_4H_9\text{-t})Si(CH_3)_2$ | E15 |
| 2.39 | $C_4H_9$-t | H | $(C_4H_9\text{-t})Si(CH_3)_2$ | E3 |
| 2.40 | $C_4H_9$-t | H | H | E4 |
| 2.41 | Phenyl | H | $(C_4H_9\text{-t})Si(CH_3)_2$ | E5 |
| 2.42 | Phenyl | H | H | E6 |
| 2.43 | $C_3H_7$-iso | $C_3H_7$-iso | $(C_4H_9\text{-t})Si(CH_3)_3$ | E7 |
| 2.44 | $C_3H_7$-iso | $C_3H_7$-iso | H | E8 |
| 2.45 | Cyclohexyl | H | $(C_4H_9\text{-t})Si(CH_3)_2$ | E11 |
| 2.46 | Cyclohexyl | H | H | E12 |
| 2.47 | $C_6H_4Br(4)$ | H | $(C_4H_9\text{-t})Si(CH_3)_2$ | E13 |

Fortsetzung von Tabelle 2:

| Verb. Nr. | $R_3$ | $R_4$ | $R_1$ | Physik.Konst. bzw. Beispiel |
|---|---|---|---|---|
| 2.48 | $C_6H_4NO_2(4)$ | H | $(C_4H_9-t)Si(CH_3)_2$ | E19 |
| 2.49 | Benzyl | H | $(C_4H_9-t)Si(CH_3)_2$ | E27 |
| 2.50 | $C_6H_4CF_3(2)$ | H | $(C_4H_9-t)Si(CH_3)_2$ | E21 |
| 2.51 | $C_6H_4CF_3(2)$ | H | H | E21 |
| 2.52 | $CCl_3$ | H | $(C_4H_9-t)Si(CH_3)_2$ | E22 |
| 2.53 | 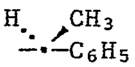 | H | H | E30 |
| 2.54 | 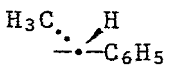 | H | $(C_4H_9-t)Si(CH_3)_2$ | E29 |
| 2.55 | 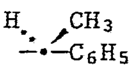 | H | $(C_4H_9-t)Si(CH_3)_2$ | E31 |
| 2.56 | 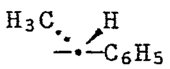 | H | H | E32 |
| 2.57 | $C_6H_4Br_2(2)$ | H | $(C_4H_9-t)Si(CH_3)_2$ | E33 |
| 2.58 | $C_6H_4Br_2(2)$ | H | H | E34 |
| 2.59 | $C_6H_4(C_2H_5)(2)$ | H | $(C_4H_9-t)Si(CH_3)_2$ | E35 |
| 2.60 | $C_6H_4(C_2H_5)(2)$ | H | H | E36 |
| 2.61 | $C_6H_4NO_2(2)$ | H | $(C_4H_9-t)Si(CH_3)_2$ | E37 |
| 2.62 | $C_6H_4NO_2(2)$ | H | H | E38 |
| 2.63 | $C_6H_3(CH_3)_2(2,6)$ | H | $(C_4H_9-t)Si(CH_3)_2$ | E39 |
| 2.64 | $C_6H_3(CH_3)_2(2,6)$ | H | H | E40 |
| 2.65 | $CH_2CH_2Cl$ | H | $(C_4H_9-t)Si(CH_3)_2$ | E41 |
| 2.66 | 1-Naphthyl | H | $(C_4H_9-t)Si(CH_3)_2$ | E43 |
| 2.67 | 1-Naphthyl | H | H | E44 |

Tabelle 3: Typische Vertreter von Milbemycin-D-derivaten der Formel I ($R_2$ = $C_3H_7$-iso):

| Verb. Nr. | $R_3$ | $R_4$ | $R_1$ | Physik.Konst. bzw. Beispiel |
|---|---|---|---|---|
| 3.1 | $CH_3$ | H | H | E10 |
| 3.2 | $C_2H_5$ | H | H | |
| 3.3 | $C_3H_7$-n | H | H | |
| 3.4 | $C_3H_7$-iso | H | H | |
| 3.5 | $C_4H_9$-n | H | H | |
| 3.6 | $CH_2CH_2Cl$ | H | H | |
| 3.7 | Cyclohexyl | H | H | |
| 3.8 | $CH_2CH=CH_2$ | H | H | |
| 3.9 | 1-Cyclohexan-1-yl | H | H | |
| 3.10 | Benzyl | H | H | |
| 3.11 | $C_6H_4Br(4)$ | H | H | |
| 3.12 | $C_6H_4Cl(3)$ | H | H | |
| 3.13 | $C_6H_4F(2)$ | H | H | |
| 3.14 | $C_6H_3Cl_2(3,4)$ | H | H | |
| 3.15 | $C_6H_3F_2(2,4)$ | H | H | |
| 3.16 | $C_6H_4CH_3(4)$ | H | H | |
| 3.17 | $C_6H_4OC_2H_5(2)$ | H | H | |
| 3.18 | $C_6H_4OCH_3(3)$ | H | H | |
| 3.19 | $C_6H_4NO_2(4)$ | H | H | |
| 3.20 | $C_6H_3F(4)NO_2(3)$ | H | H | |
| 3.21 | $CH_3$ | $CH_3$ | H | |
| 3.22 | $C_2H_5$ | $C_2H_5$ | H | |
| 3.23 | $C_3H_7$-n | $C_3H_7$-n | H | |
| 3.24 | $C_3H_7$-iso | $C_3H_7$-iso | H | |
| 3.25 | $C_4H_9$-tert | $C_4H_9$-tert | H | |
| 3.26 | $C_4H_9$-sek | $C_4H_9$-sek | H | |
| 3.27 | Phenyl | Phenyl | H | |
| 3.28 | $C_6H_{13}$-n | H | H | |
| 3.29 | $CH_3$ | H | $(C_4H_9\text{-t})Si(CH_3)_2$ | E9 |

Fortsetzung von Tabelle 3:

| Verb. Nr. | $R_3$ | $R_4$ | $R_1$ |
|---|---|---|---|
| 3.30 | $C_2H_5$ | H | $(C_4H_9\text{-t})Si(CH_3)_2$ |
| 3.31 | $C_3H_7$-n | H | $(C_4H_9\text{-t})Si(CH_3)_2$ |
| 3.32 | $C_3H_7$-iso | H | $(C_4H_9\text{-t})Si(CH_3)_2$ |
| 3.33 | $C_4H_9$-n | H | $(C_4H_9\text{-t})Si(CH_3)_2$ |
| 3.34 | $C_6H_4F(2)$ | H | $(C_4H_9\text{-t})Si(CH_3)_2$ |
| 3.35 | $CH_3$ | H | $(C_4H_9\text{-t})Si(C_6H_5)_2$ |
| 3.36 | $C_2H_5$ | H | $(C_3H_7\text{-iso})_2SiCH_3$ |
| 3.37 | $C_3H_7$-n | H | $Si(C_6H_5)_3$ |
| 3.38 | $CH_3$ | $CH_3$ | $(C_4H_9\text{-t})Si(CH_3)_2$ |

Tabelle 4: Typische Vertreter von 13-Deoxi-22,23-dihydro-C-076-B1A-aglycon-derivaten der Formel I ($R_2$ = $C_4H_9$-sek):

| Verb. Nr. | $R_3$ | $R_4$ | $R_1$ |
|---|---|---|---|
| 4.1 | $CH_3$ | H | H |
| 4.2 | $C_2H_5$ | H | H |
| 4.3 | $C_3H_7$-n | H | H |
| 4.4 | $C_3H_7$-iso | H | H |
| 4.5 | $C_4H_9$-n | H | H |
| 4.6 | $CH_2CH_2Cl$ | H | H |
| 4.7 | Cyclohexyl | H | H |
| 4.8 | $CH_2CH{=}CH_2$ | H | H |
| 4.9 | 1-Cyclohexan-1-yl | H | H |
| 4.10 | Benzyl | H | H |
| 4.11 | $C_6H_4Br(4)$ | H | H |
| 4.12 | $C_6H_4Cl(3)$ | H | H |
| 4.13 | $C_6H_4F(2)$ | H | H |
| 4.14 | $C_6H_3Cl_2(3,4)$ | H | H |
| 4.15 | $C_6H_3F_2(2,4)$ | H | H |

Fortsetzung von Tabelle 4:

| Verb. Nr. | $R_3$ | $R_4$ | $R_1$ |
|---|---|---|---|
| 4.16 | $C_6H_4CH_3(4)$ | H | H |
| 4.17 | $C_6H_4OC_2H_5(2)$ | H | H |
| 4.18 | $C_6H_4OCH_3(3)$ | H | H |
| 4.19 | $C_6H_4NO_2(4)$ | H | H |
| 4.20 | $C_6H_3F(4)NO_2(3)$ | H | H |
| 4.21 | $CH_3$ | $CH_3$ | H |
| 4.22 | $C_2H_5$ | $C_2H_5$ | H |
| 4.23 | $C_3H_7$-n | $C_3H_7$-n | H |
| 4.24 | $C_3H_7$-iso | $C_3H_7$-iso | H |
| 4.25 | $C_4H_9$-tert | $C_4H_9$-tert | H |
| 4.26 | $C_4H_9$-sek | $C_4H_9$-sek | H |
| 4.27 | Phenyl | Phenyl | H |
| 4.28 | $C_6H_{13}$-n | H | H |
| 4.29 | $CH_3$ | H | $(C_4H_9\text{-t})Si(CH_3)_2$ |
| 4.30 | $C_2H_5$ | H | $(C_4H_9\text{-t})Si(CH_3)_2$ |
| 4.31 | $C_3H_7$-n | H | $(C_4H_9\text{-t})Si(CH_3)_2$ |
| 4.32 | $C_3H_7$-iso | H | $(C_4H_9\text{-t})Si(CH_3)_2$ |
| 4.33 | $C_4H_9$-n | H | $(C_4H_9\text{-t})Si(CH_3)_2$ |
| 4.34 | $C_6H_4F(2)$ | H | $(C_4H_9\text{-t})Si(CH_3)_2$ |
| 4.35 | $CH_3$ | H | $(C_4H_9\text{-t})Si(C_6H_5)_2$ |
| 4.36 | $C_2H_5$ | H | $(C_3H_7\text{-iso})_2SiCH_3$ |
| 4.37 | $C_3H_7$-n | H | $Si(C_6H_5)_3$ |
| 4.38 | $CH_3$ | $CH_3$ | $(C_4H_9\text{-t})Si(CH_3)_2$ |

Formulierungsbeispiele für den Wirkstoff der Formel I

(% = Gewichtsprozent)

| Spritzpulver | a) | b) | c) |
|---|---|---|---|
| Wirkstoff Nr. E1 bis E14 | 25 % | 50 % | 75 % |
| Na-Ligninsulfonat | 5 % | 5 % | - |
| Na-Laurylsulfat | 3 % | - | 5 % |

| | | | |
|---|---|---|---|
| Na-Diisobutylnaphthalinsulfonat | - | 6 % | 10 % |
| Octylphenolpolyethylenglykol-ether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

Emulsions-Konzentrat

Wirkstoff Nr. E1 bis E46 10 %
Octylphenolpolyethylenglykolether (4-5 Mol AeO) 3 %
Ca-Dodecylbenzolsulfonat 3 %
Ricinusölpolyglykolether (36 Mol AeO) 4 %
Cyclohexanon 30 %
Xylolgemisch 50 %

Aus diesem Konzentrat können durch Verdünnen mit Wasser Emulsionen jeder gewünschten Konzentration hergestellt werden.

| Stäubemittel | a) | b) |
|---|---|---|
| Wirkstoff Nr. E1 bis E46 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Extruder Granulat

Wirkstoff Nr. E1 bis E46 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert und anschliessend im Luftstrom getrocknet.

Tabellen bzw. Boli

I Ein Wirkstoff E1 bis E46 33,0 %
Methylcellulose 0,80 %
Kieselsäure hochdispers 0,80 %
Maisstärke 8,40 %

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

II Milchzucker krist. 22,50 %
Maisstärke 17,00 %
mikrokrist. Cellulose 16,50 %
Magnesiumstearat 1,00 %

Alle 4 Hilfsstoffe gut mischen

Phasen I und II mischen und zu Tabletten oder Boli verpressen.

Falls die Verbindung der Formel I bzw. entsprechenden Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden

Mittel können auch dem Futter oder den Tränken zuge setzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die enthaltende Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

Biologische Beispiele

B-1. Insektizide Frassgift-Wirkung bei Spodoptera littoralis

Eingetopfe Baumwollpflanzen im 5-Blatt-Stadium werden mit einer acetonisch/wässrigen Versuchslösung besprüht, die 3, 12,5 oder 50 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch wird bei ca. 24° C und 60 % relativer Luftfeuchtigkeit durchgeführt Auswertungen und Zwischenauswertungen auf moribunde Tiere, Wachstum und Larven und Frasschäden erfolgen nach 24 Stunden, 48 Stunden und 72 Stunden.

Die Verbindungen der Formeln I aus den Herstellungsbeispielen, wie z.B. die Verbindungen gemäss den Herstellungsbeispielen E10, E6, E4, E14, E12, E16 und E22, erzielten bereits bei einer Wirkstoffkonzentration von 12,5 ppm eine vollständige Abtötung nach 24 Stunden.

B-2. Wirkung gegen pflanzenschädigende Akariden OP-sensible Tetranychus urticae

Die Primärblätter von Bohnenpflanzen (Phaseolus vulgaris) werden 16 Stunden vor dem Versuch mit einem durch T. urticae infestierten, aus einer Massenzucht stammenden Blattstück belegt. Die so mit allen Milben-Stadien infestierten Pflanzen werden nach Entfernung des Blattstücks mit einer Versuchslösung bis zur Tropfnässe besprüht, die wahlweise 0,4 ppm oder 1,6 ppm der zu prüfenden Verbindung enthält. Die Temperatur in der Gewächshauskabine beträgt ca. 25° C.

Nach sieben Tagen wird unter dem Binokular auf Prozentsatz mobiler Stadien (Adulte und Nymphen) und auf vorhandene Eier ausgewertet.

Verbindungen der Formel I wie die aus den Herstellungsbeispielen E1 bis E14 sowie E20 und E22 erzielten bei einer Wirkstoffkonzentration von 1,6 ppm vollständige Abtötung.

B-3. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca. 50° C vermischt, dass ein Homogenisat von wahlweise 250 ppm oder 125 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Die Verbindungen aus den Herstellungsbeispielen zeigen bei 250 ppm eine Wirkung von 100 %, wobei die Verbindungen aus den Herstellungsbeispiele, wie z.B. E8, E6, E2, E14, E22, E28, E26, E30, E32, E34, E36 und E38, diese Wirkung auch noch bei der reduzierten Wirkstoffkonzentration von 100 ppm erzielten.

B-4. Milbizide Wirkung gegen Dermanyssus gallinae

2 bis 3 ml einer Testlösung (100, 10, 1 und 0,1 ppm Aktivsubstanz) werden in einem nach oben offenen Glasbehälter gegeben und ca. 200 Milben in unterschiedlichen Entwicklungstadien in diesen Behälter eingesetzt. Der Glasbehälter wird mit einem Wattebausch verschlossen und 10 Minuten gleichmässig, bis zur vollständigen Benetzung der Milben geschüttelt. Danach wird der Behälter umgekehrt hingestellt, bis die überschüssige Testlösung von der Watte aufgesogen ist. Der Behälter wird erneut gekehrt und die behandelten Zecken zur Evaluierung der Wirksamkeit der Testsubstanzen drei Tage unter Laborbedingungen beobachtet, wobei die Mortalität als Massstab für Wirksamkeit herangezogen wird.

Verbindungen aus den Herstellungsbeispielen, wie. z.B. E10, E8 und E26 zeigen bei 10 ppm eine Wirkung von 100%.

B-5. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus concortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 1 mg oder 0,5 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen der Formeln I bei 1 mg/kg behandelt wurden, zeigten im Vergleich zu unbehandelten, aber infizierten Vergleichsgruppen keinen Nematodenbefall (= komplette Reduktion der Wurmeier im Kot). Verbindungen aus den Herstellungsbeispielen, wie. z.B. E4, E8, E14, E18, E30 bis E32, E 34 und E36, bewirkten

dies auch noch bei 0,5 mg/kg.

B-6. Kontaktwirkung auf Aphis craccivora

Erbsenkeimlinge, die mit sämtlichen Entwicklungsstadien der Laus infiziert sind, werden mit einer aus einem Emulsionskonzentrat hergestellten Wirkstofflösung besprüht, die wahlweise 50 ppm, 25 ppm oder 12,5 ppm Wirkstoff enthält. Nach 3 Tagen wird auf mehr als 80 % tote bzw. abgefallene Blattläuse ausgewertet. Nur bei dieser Wirkungshöhe wird ein Präparat als wirksam eingestuft.

Verbindungen aus den Herstellungsbeispielen, wie z.B. E10, E8, E14, E12 und E22, erzielten bei einer Konzentration von 25 ppm eine vollständige Abtötung (= 100 %).

B-7. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Verbindungen aus den Herstellungsbeispielen, wie. z.B. E4, E8, E14, E20 und E22 bewirkten in diesem Test bei einer Konzentration von 3,3 ppm bereits nach einem Tag eine vollständige Abtötung sämtlicher Larven.

B-8. Bodennematizide Wirkung gegen Meloidogyne incognita

Eier von Meloidogyne incognita werden in Sand eingemischt. Dieses Gemisch wird dann in 200 ml fassende Tontöpfe eingebracht (5000 Eier pro Topf). Am gleichen Tag wird pro Topf eine 3 Wochen alte Tomatenpflanze gepflanzt und der formulierte Wirkstoffe mittels Druckapplikation in die Töpfe hineingegeben (0,002 % Aktivsubstanz bezogen auf das Bodenvolumen). Die eingetopften Pflanzen werden nun bei einer Temperatur von 26±1°C und einer relativen Luftfeuchtigkeit von 60 % in einem Gewächshaus aufgestellt. Nach Ablauf von 4 Wochen erfolgt eine Evaluierung durch Untersuchung der Pflanzen auf Wurzelgallbildung nach dem sogenannten Wurzelgall-Index ("Knot Index").

Die Verbindungen der Herstellungsbeispiele zeigen gegen Meloidogyne incognita durch weitgehende Reduktion der Wurzelgallbildung gute Wirksamkeit. Unbehandelte jedoch infizierte Kontrollpflanzen weisen dagegen starke Wurzelgallbildung auf (= 100 %). So hemmen z.B. die Verbindungen aus den Herstellungsbeispielen im obigen Versuch die Wurzelgallbildung fast vollständig (0-10 % Restbefall). Als besonders aktiv erweisen sich die Verbindung z.B. gemäss den Herstellungsbeispielen E2, E10, E16 und E18.

## Patentansprüche

1. Verbindungen der Formel I

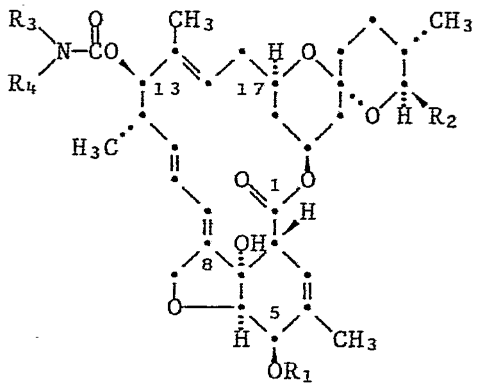

(I)

in welcher

$R_1$ Wasserstoff, eine Silyl- oder Acylgruppe,

$R_2$ Methyl, Ethyl, Isopropyl oder sek.Butyl,

$R_3$ eine unsubstituierte oder substituierte Gruppe ausgewählt aus der Reihe geradkettiges oder verzweigtes $C_1$-$C_{18}$-Alkyl, $C_3$-$C_{10}$-Cycloalkyl, $C_2$-$C_6$-Alkenyl, $C_3$-$C_{10}$-Cycloalkenyl, Phenyl und Naphthyl, und

$R_4$ Wasserstoff oder einen unter $R_3$ angegebenen Rest bedeuten.

2. Verbindungen der Formel I nach Anspruch 1, worin

$R_1$ Wasserstoff darstellt,

$R_2$ für Methyl, Ethyl, Isopropyl oder sek.Butyl steht,

$R_3$ unsubstituiertes oder durch Halogen, $C_1$-$C_4$-Alkoxy, Acyloxy oder eine unsubstituierte oder durch Halogen, $C_1$-$C_2$-Alkyl, $C_1$-$C_2$-Alkoxy, $C_1$-$C_2$-Haloalkyl oder Nitro substituierte Phenyl- oder Naphthylgrup-

pe substituiertes $C_1$-$C_{18}$-Alkyl, unsubstituiertes oder durch Halogen und/oder $C_1$-$C_4$-Alkyl substituiertes $C_3$-$C_{10}$-Cycloalkyl, unsubstituiertes oder durch Halogen substituiertes $C_2$-$C_6$-Alkenyl, unsubstituiertes oder durch Halogen und/oder $C_1$-$C_4$-Alkyl substituiertes $C_3$-$C_{10}$-Cycloalkenyl darstellt, und
$R_4$ für Wasserstoff steht.

3. Verbindungen der Formel I nach Anspruch 2, worin
$R_1$ Wasserstoff darstellt,
$R_2$ für Methyl, Ethyl, Isopropyl oder sek.Butyl steht, und
$R_3$ unsubstituiertes oder durch Halogen, Phenyl oder Naphthyl substituiertes $C_1$-$C_8$-Alkyl, $C_5$-$C_7$-Cycloalkyl, $C_2$-$C_6$-Alkenyl und $C_5$-$C_7$-Cycloalkenyl darstellt, und
$R_4$ für Wasserstoff steht.

4. Verbindungen der Formel I nach Anspruch 2, worin
$R_1$ Wasserstoff darstellt,
$R_2$ für Methyl, Aethyl, Isopropyl oder sek.Butyl steht,
$R_3$ unsubstituiertes oder durch Halogen, $C_1$-$C_6$-Alkyl, $C_1$-$C_6$-Alkoxy, $C_1$-$C_4$-Alkylthio, $C_1$-$C_4$-Haloalkyl oder Nitro substituiertes Phenyl oder Naphthyl darstellt, und
$R_4$ für Wasserstoff steht.

5. Verbindungen der Formel I nach Anspruch 4, worin
$R_1$ Wasserstoff darstellt,
$R_2$ für Methyl, Aethyl, Isopropyl oder sek.Butyl steht, und
$R_3$ unsubstituiertes oder durch Brom, Chlor, Fluor, $C_1$-$C_2$-Alkyl, $C_1$-$C_2$-Alkoxy, $C_1$-$C_2$-Haloalkyl oder Nitro substituiertes Phenyl oder Naphthyl darstellt, und
$R_4$ für Wasserstoff steht.

6. Verbindungen der Formel I nach Anspruch 5, worin
$R_1$ Wasserstoff darstellt,
$R_2$ für Methyl, Ethyl, Isopropyl oder sek. Butyl steht, und
$R_3$ unsubstituiertes oder durch Brom, Chlor, Fluor, $C_1$-$C_2$-Alkyl, $C_1$-$C_2$-Alkoxy, Trifluormethyl oder Nitro substituiertes Phenyl oder unsubstituiertes Naphthyl darstellt.

7. Verbindungen der Formel I nach Anspruch 2, worin
$R_1$ Wasserstoff darstellt,
$R_2$ für Methyl, Ethyl, Isopropyl oder sek.Butyl steht, und
$R_3$ unsubstituiertes oder durch Halogen, $C_1$-$C_4$-Alkoxy, oder $C_1$-$C_4$-Acyloxy substituiertes $C_1$-$C_8$-Alkyl, unsubstituiertes oder durch Halogen, $C_1$-$C_6$-Alkyl, $C_1$-$C_4$-Alkylthio, $C_1$-$C_4$-Haloalkyl oder Nitro substituiertes Phenyl darstellt, und
$R_4$ einen unter $R_3$ angegebenen Rest bedeutet.

8. Verbindungen der Formel I nach Anspruch 7, worin
$R_1$ Wasserstoff darstellt,
$R_2$ für Methyl, Ethyl, Isopropyl oder sek.Butyl steht, und
$R_3$ $C_1$-$C_8$-Alkyl, oder Phenyl darstellt, und
$R_4$ einen unter $R_3$ angegebenen Rest bedeutet.

9. Eine Verbindung der Formel I nach Anspruch 1, ausgewählt aus der Reihe:
13β-N-Methylcarbamoyloxy-milbemycin D,
13β-N-Methylcarbamoyloxy-milbemycin $A_3$,
13β-N-Methylcarbamoyloxy-milbemycin $A_4$,
13β-N-tert.-Butylcarbamoyloxy-milbemycin D,
13β-N-tert.-Butylcarbamoyloxy-milbemycin $A_3$,
13β-N-tert.-Butylcarbamoyloxy-milbemycin $A_4$,
13β-N-Phenylcarbamoyloxy-milbemycin D,
13β-N-Phenylcarbamoyloxy-milbemycin $A_3$,
13β-N-Phenylcarbamoyloxy-milbemycin $A_4$,
13β-N,N-Dimethylcarbamoyloxy-milbemycin D,
13β-N,N-Dimethylcarbamoyloxy-milbemycin $A_3$,
13β-N,N-Dimethylcarbamoyloxy-milbemycin $A_4$,
13β-N,N-Diisopropylcarbamoyloxy-milbemycin D,
13β-N,N-Diisopropylcarbamoyloxy-milbemycin $A_3$ und
13β-N,N-Diisopropylcarbamoyloxy-milbemycin $A_4$.

10. Eine Verbindung der Formel I nach Anspruch 1, ausgewählt aus der Reihe:
5-O-tert.-Butyldimethylsilyl-13β-N-methylylcarbamoyloxy-milbemycin D,
5-O-tert.-Butyldimethylsilyl-13β-N-methylylcarbamoyloxy-milbemycin $A_3$,
5-O-tert.-Butyldimethylsilyl-13β-N-methylylcarbamoyloxy-milbemycin $A_4$,
5-O-tert.-Butyldimethylsilyl-13β-N-tert.-butylcarbamoyloxy-milbemycin D,
5-O-tert.-Butyldimethylsilyl-13β-N-tert.-butylcarbamoyloxy-milbemycin $A_3$,
5-O-tert.-Butyldimethylsilyl-13β-N-tert.-butylcarbamoyloxy-milbemycin $A_4$,
5-O-tert.-Butyldimethylsilyl-13β-N-phenylcarbamoyloxy-milbemycin D,
5-O-tert.-Butyldimethylsilyl-13β-N-phenylcarbamoyloxy-milbemycin $A_3$,
5-O-tert.-Butyldimethylsilyl-13β-N-phenylcarbamoyloxy-milbemycin $A_4$,

5-O-tert.-Butyldimethylsilyl-13β-N,N-dimethylcarbamoyloxy-milbemycin D,
5-O-tert.-Butyldimethylsilyl-13β-N,N-dimethylcarbamoyloxy-milbemycin $A_3$,
5-O-tert.-Butyldimethylsilyl-13β-N,N-dimethylcarbamoyloxy-milbemycin $A_4$,
5-O-tert.-Butyldimethylsilyl-13β-N,N-diisopropylcarbamoyloxy-milbemycin D,
5-O-tert.-Butyldimethylsilyl-13β-N,N-diisopropylcarbamoyloxy-milbemycin $A_3$ und
5-O-tert.-Butyldimethylsilyl-13β-N,N-diisopropylcarbamoyloxy-milbemycin $A_4$.

11. Verfahren zur Herstellung von Verbindungen der Formel I

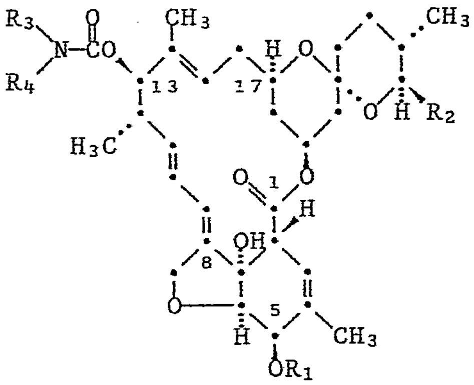

(I)

in welcher

$R_1$ Wasserstoff, eine Silyl- oder Acylgruppe,

$R_2$ Methyl, Ethyl, Isopropyl oder sek.Butyl,

$R_3$ eine unsubstituierte oder substituierte Gruppe ausgewählt aus der Reihe geradkettiges oder verzweigtes $C_1$-$C_{18}$-Alkyl, $C_3$-$C_{10}$-Cycloalkyl, $C_2$-$C_6$-Alkenyl, $C_3$-$C_{10}$-Cycloalkenyl, Phenyl und Naphthyl, und

$R_4$ Wasserstoff oder einen unter $R_3$ angegebenen Rest bedeuten, dadurch gekennzeichnet, dass man

A) in den Fällen, in denen eine Verbindung der Formel I hergestellt werden soll, worin $R_4$ für Wasserstoff steht, wird ein Allylalkohol der Formel II

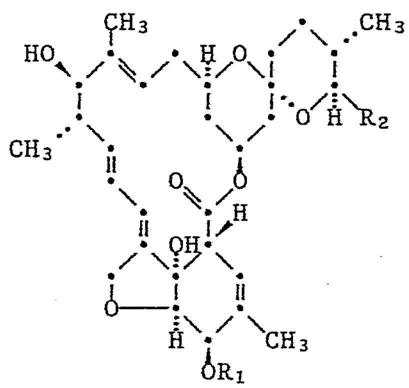

(II),

worin $R_1$ eine Schutzgruppe bedeutet und $R_2$ die unter Formel I angegebene Bedeutung besitzt, mit einem zur Einführung einer 13β-Carbamoyloxygruppe $R_3NH$-C(O)O- geeigneten Reagenz behandelt und danach, sofern ein Produkt mit einer freien 5-Hydroxy-Gruppe erwünscht ist, die Schutzgruppe $R_1$ hydrolytisch abgespaltet, oder

B) in den Fällen, in denen eine Verbindung der Formel I hergestellt werden soll, worin $R_4$ eine der unter $R_3$ angegebenen Bedeutungen hat, wird ein Allylalkohol der Formel II, worin $R_1$ eine Schutzgruppe bedeutet und $R_2$ die unter Formel I angegebene Bedeutung besitzt, mit einem zur Einführung einer 13β-Carbamoyloxygruppe

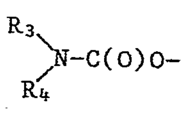

($R_4 \neq$ H) geeigneten Reagenz behandelt und danach, sofern ein Produkt mit einer freien 5-Hydroxy-Gruppe erwünscht ist, die Schutzgruppe $R_1$ hydrolytisch abgespaltet, oder

C) durch Umsetzung einer Verbindung der Formel V,

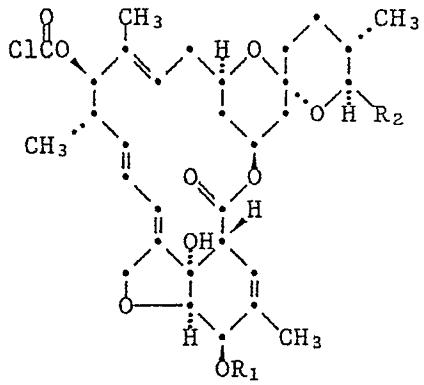

(V),

worin $R_1$ für eine Schutzgruppe steht und $R_2$ eine der unter Formel I angegebenen Bedeutungen hat, mit einem primären oder sekundären Amin der Formel VI,

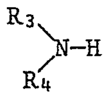

(VI),

worin $R_3$ und $R_4$ wie unter Formel I definiert sind, erhalten werden und sofern ein Produkt mit einer freien 5-Hydroxy-Gruppe erwünscht ist, durch anschliessende hydrolytische Abspaltung der Schutzgruppe $R_1$.

12. Pestizides Mittel dadurch gekennzeichnet, dass es neben üblichen Formulierungshilfsstoffen als Wirkkomponente mindestens eine Verbindung der Formel I

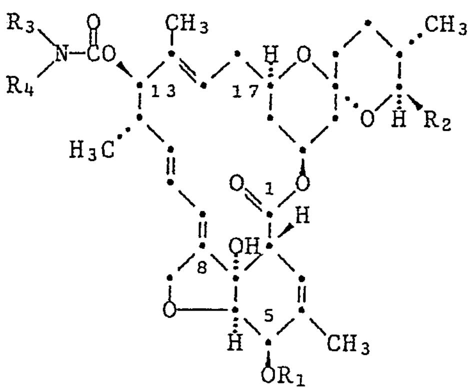

(I)

in welcher
$R_1$ Wasserstoff, eine Silyl- oder Acylgruppe,
$R_2$ Methyl, Ethyl, Isopropyl oder sek.Butyl,
$R_3$ eine unsubstituierte oder substituierte Gruppe ausgewählt aus der Reihe geradkettiges oder verzweigtes $C_1$-$C_{18}$-Alkyl, $C_3$-$C_{10}$-Cycloalkyl, $C_2$-$C_6$-Alkenyl, $C_3$-$C_{10}$-Cycloalkenyl, Phenyl und Naphthyl, und
$R_4$ Wasserstoff oder einen unter $R_3$ angegebenen Rest bedeuten, enthält.

13. Pestizides Mittel nach Anspruch 12, dadurch gekennzeichnet, dass es neben üblichen Formulierungshilfsstoffen als Wirkkomponente mindestens eine Verbindung der Formel I gemäss einem der Ansprüche 2 bis 10 enthält.

14. Verbindung der Formel I

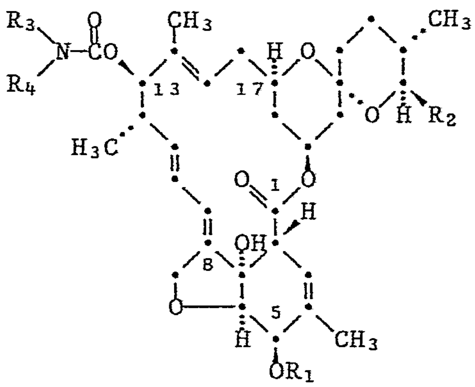

(I)

in welcher
$R_1$ Wasserstoff, eine Silyl- oder Acylgruppe,
$R_2$ Methyl, Ethyl, Isopropyl oder sek.Butyl,
$R_3$ eine unsubstituierte oder substituierte Gruppe ausgewählt aus der Reihe geradkettiges oder verzweigtes $C_1$-$C_{18}$-Alkyl, $C_3$-$C_{10}$-Cycloalkyl, $C_2$-$C_6$-Alkenyl, $C_3$-$C_{10}$-Cycloalkenyl, Phenyl und Naphthyl, und
$R_4$ Wasserstoff oder einen unter $R_3$ angegebenen Rest bedeuten, zur Bekämpfung von Insekten, von Schädlingen der Ordnung Acarina, oder von Nematoden.

15. Verwendung nach Anspruch 14, dadurch gekennzeichnet, dass man eine Verbindung der Formel I nach einem der Ansprüche 2 bis 10 einsetzt.

16. Verwendung nach einem der Ansprüche 14 oder 15, dadurch gekennzeichnet, dass es sich bei den Schädlingen um Ekto- oder Endoparasiten bei Nutztieren handelt.

17. Verfahren zur Bekämpfung von tierparasitären oder pflanzenparasitären Schädlingen, dadurch gekennzeichnet, dass man eine Verbindung der Formel I

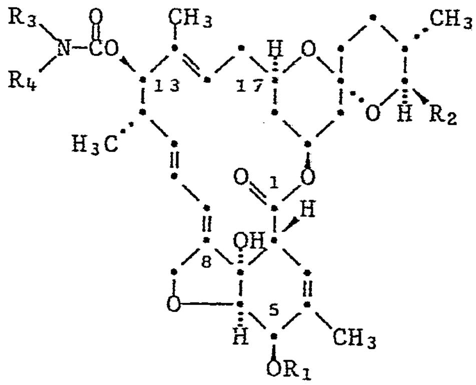

(I)

in welcher
$R_1$ Wasserstoff, eine Silyl- oder Acylgruppe,
$R_2$ Methyl, Ethyl, Isopropyl oder sek.Butyl,
$R_3$ eine unsubstituierte oder substituierte Gruppe ausgewählt aus der Reihe geradkettiges oder verzweigtes $C_1$-$C_{18}$-Alkyl, $C_3$-$C_{10}$-Cycloalkyl, $C_2$-$C_6$-Alkenyl, $C_3$-$C_{10}$-Cycloalkenyl, Phenyl und Naphthyl, und
$R_4$ Wasserstoff oder einen unter $R_3$ angegebenen Rest bedeuten, auf den Parasiten, das Wirtstier, die Pflanze oder den Lebensraum des Parasiten appliziert.

18. Verfahren nach Anspruch 17, dadurch gekennzeichnet, dass man bei dem Verfahren eine Verbindung der Formel I gemäss einem der Ansprüche 2 bis 10 einsetzt.

Patentansprüche für den folgenden Vertragsstaat: AT

1. Verfahren zur Herstellung von Verbindung der Formel I

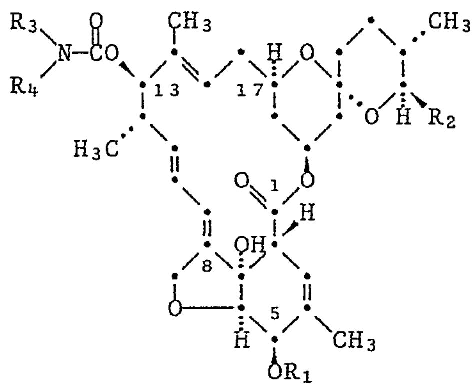

(I)

in welcher
$R_1$ Wasserstoff, eine Silyl- oder Acylgruppe,
$R_2$ Methyl, Ethyl, Isopropyl oder sek.Butyl,
$R_3$ eine unsubstituierte oder substituierte Gruppe ausgewählt aus der Reihe geradkettiges oder verzweigtes $C_1$-$C_{18}$-Alkyl, $C_3$-$C_{10}$-Cycloalkyl, $C_2$-$C_6$-Alkenyl, $C_3$-$C_{10}$-Cycloalkenyl, Phenyl und Naphthyl, und
$R_4$ Wasserstoff oder einen unter $R_3$ angegebenen Rest bedeuten, dadurch gekennzeichnet, dass man

A) in den Fällen, in denen eine Verbindung der Formel I hergestellt werden soll, worin $R_4$ für Wasserstoff steht, wird ein Allylalkohol der Formel II

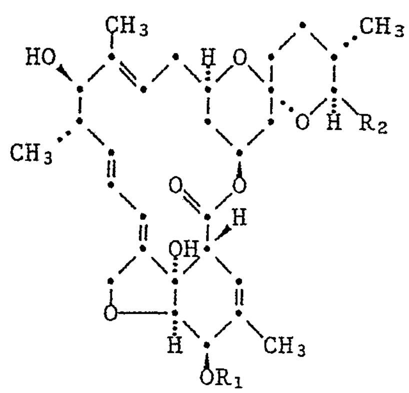

(II),

worin $R_1$ eine Schutzgruppe bedeutet und $R_2$ die unter Formel I angegebene Bedeutung besitzt, mit einem zur Einführung einer 13β-Carbamoyloxygruppe $R_3NH$-C(O)O- geeigneten Reagenz behandelt und danach, sofern ein Produkt mit einer freien 5-Hydroxy-Gruppe erwünscht ist, die Schutzgruppe $R_1$ hydrolytisch abgespaltet, oder

B) in den Fällen, in denen eine Verbindung der Formel I hergestellt werden soll, worin $R_4$ eine der unter $R_3$ angegebenen Bedeutungen hat, wird ein Allylalkohol der Formel II, worin $R_1$ eine Schutzgruppe bedeutet und $R_2$ die unter Formel I angegebene Bedeutung besitzt, mit einem zur Einführung einer 13β-Carbamoyloxygruppe

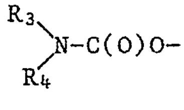

($R_4 \neq H$) geeigneten Reagenz behandelt und danach, sofern ein Produkt mit einer freien 5-Hydroxy-Gruppe erwünscht ist, die Schutzgruppe $R_1$ hydrolytisch abgespaltet, oder

C) durch Umsetzung einer Verbindung der Formel V,

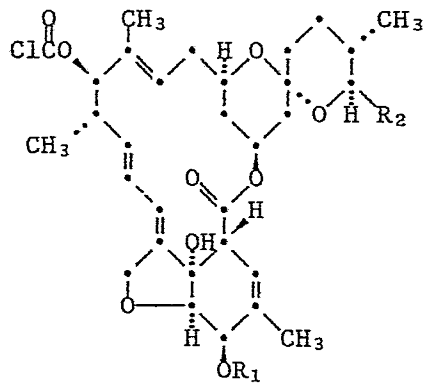

(V),

worin $R_1$ für eine Schutzgruppe steht und $R_2$ eine der unter Formel I angegebenen Bedeutungen hat, mit einem primären oder sekundären Amin der Formel VI,

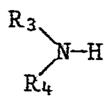

(VI),

worin $R_3$ und $R_4$ wie unter Formel I definiert sind, erhalten werden und sofern ein Produkt mit einer freien 5-Hydroxy-Gruppe erwünscht ist, durch anschliessende hydrolytische Abspaltung der Schutzgruppe $R_1$.

2. Pestizides Mittel dadurch gekennzeichnet, dass es neben üblichen Formulierungshilfsstoffen als Wirkkomponente mindestens eine Verbindung der Formel I

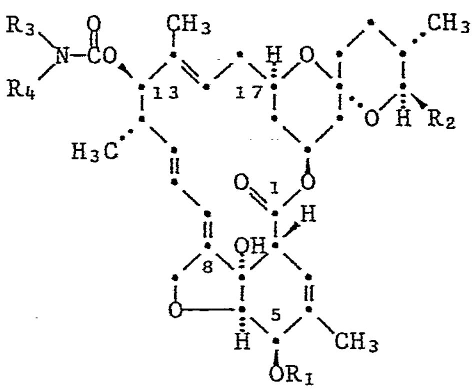

(I)

in welcher
$R_1$ Wasserstoff, eine Silyl- oder Acylgruppe,
$R_2$ Methyl, Ethyl, Isopropyl oder sek.Butyl,
$R_3$ eine unsubstituierte oder substituierte Gruppe ausgewählt aus der Reihe geradkettiges oder verzweigtes $C_1$-$C_{18}$-Alkyl, $C_3$-$C_{10}$-Cycloalkyl, $C_2$-$C_6$-Alkenyl, $C_3$-$C_{10}$-Cycloalkenyl, Phenyl und Naphthyl, und
$R_4$ Wasserstoff oder einen unter $R_3$ angegebenen Rest bedeuten, enthält.

3. Mittel nach Anspruch 2, enthaltend als Wirkstoff eine der Verbindungen der Formel I, worin
$R_1$ Wasserstoff darstellt,
$R_2$ für Methyl, Ethyl, Isopropyl oder sek.Butyl steht,
$R_3$ unsubstituiertes oder durch Halogen, $C_1$-$C_4$-Alkoxy, Acyloxy oder eine unsubstituierte oder durch Halogen, $C_1$-$C_2$-Alkyl, $C_1$-$C_2$-Alkoxy, $C_1$-$C_2$-Haloalkyl oder Nitro substituierte Phenyl- oder Naphthylgruppe substituiertes $C_1$-$C_{18}$-Alkyl, unsubstituiertes oder durch Halogen und/oder $C_1$-$C_4$-Alkyl substituiertes $C_3$-$C_{10}$-Cycloalkyl, unsubstituiertes oder durch Halogen substituiertes $C_2$-$C_6$-Alkenyl, unsubstituiertes oder durch Halogen und/oder $C_1$-$C_4$-Alkyl substituiertes $C_3$-$C_{10}$-Cycloalkenyl darstellt, und
$R_4$ für Wasserstoff steht.

4. Mittel nach Anspruch 3, enthaltend als Wirkstoff eine der Verbindungen der Formel I, worin
$R_1$ Wasserstoff darstellt,
$R_2$ für Methyl, Ethyl, Isopropyl oder sek.Butyl steht, und
$R_3$ unsubstituiertes oder durch Halogen, Phenyl oder Naphthyl substituiertes $C_1$-$C_8$-Alkyl, $C_5$-$C_7$-Cycloal-

kyl, $C_2$-$C_6$-Alkenyl und $C_5$-$C_7$-Cycloalkenyl darstellt, und
$R_4$ für Wasserstoff steht.

5. Mittel nach Anspruch 3, enthaltend als Wirkstoff eine der Verbindungen der Formel I, worin
$R_1$ Wasserstoff darstellt,
$R_2$ für Methyl, Aethyl, Isopropyl oder sek.Butyl steht,
$R_3$ unsubstituiertes oder durch Halogen, $C_1$-$C_6$-Alkyl, $C_1$-$C_6$-Alkoxy, $C_1$-$C_4$-Alkylthio, $C_1$-$C_4$-Haloalkyl oder Nitro substituiertes Phenyl oder Naphthyl darstellt, und
$R_4$ für Wasserstoff steht.

6. Mittel nach Anspruch 5, enthaltend als Wirkstoff eine der Verbindungen der Formel I, worin
$R_1$ Wasserstoff darstellt,
$R_2$ für Methyl, Aethyl, Isopropyl oder sek.Butyl steht, und
$R_3$ unsubstituiertes oder durch Brom, Chlor, Fluor, $C_1$-$C_2$-Alkyl, $C_1$-$C_2$-Alkoxy, $C_1$-$C_2$-Haloalkyl oder Nitro substituiertes Phenyl oder Naphthyl darstellt, und
$R_4$ für Wasserstoff steht.

7. Mittel nach Anspruch 4, enthaltend als Wirkstoff eine der Verbindungen der Formel I, worin
$R_1$ Wasserstoff darstellt,
$R_2$ für Methyl, Ethyl, Isopropyl oder sek. Butyl steht, und
$R_3$ unsubstituiertes oder durch Brom, Chlor, Fluor, $C_1$-$C_2$-Alkyl, $C_1$-$C_2$-Alkoxy, Trifluormethyl oder Nitro substituiertes Phenyl oder unsubstituiertes Naphthyl darstellt.

8. Mittel nach Anspruch 3, enthaltend als Wirkstoff eine der Verbindungen der Formel I, worin
$R_1$ Wasserstoff darstellt,
$R_2$ für Methyl, Ethyl, Isopropyl oder sek.Butyl steht, und
$R_3$ unsubstituiertes oder durch Halogen, $C_1$-$C_4$-Alkoxy, oder $C_1$-$C_4$-Acyloxy substituiertes $C_1$-$C_8$-Alkyl, unsubstituiertes oder durch Halogen, $C_1$-$C_6$-Alkyl, $C_1$-$C_4$-Alkylthio, $C_1$-$C_4$-Haloalkyl oder Nitro substituiertes Phenyl darstellt, und
$R_4$ einen unter $R_3$ angegebenen Rest bedeutet.

9. Mittel nach Anspruch 8, enthaltend als Wirkstoff eine der Verbindungen der Formel I, worin
$R_1$ Wasserstoff darstellt,
$R_2$ für Methyl, Ethyl, Isopropyl oder sek.Butyl steht, und
$R_3$ $C_1$-$C_8$-Alkyl, oder Phenyl darstellt, und
$R_4$ einen unter $R_2$ angegebenen Rest bedeutet.

10. Mittel nach Anspruch 2, enthaltend als Wirkstoff eine Verbindung der Formel I, ausgewählt aus der Reihe:
13β-N-Methylcarbamoyloxy-milbemycin D,
13β-N-Methylcarbamoyloxy-milbemycin $A_3$,
13β-N-Methylcarbamoyloxy-milbemycin $A_4$,
13β-N-tert.-Butylcarbamoyloxy-milbemycin D,
13β-N-tert.-Butylcarbamoyloxy-milbemycin $A_3$,
13β-N-tert.-Butylcarbamoyloxy-milbemycin $A_4$,
13β-N-Phenylcarbamoyloxy-milbemycin D,
13β-N-Phenylcarbamoyloxy-milbemycin $A_3$,
13β-N-Phenylcarbamoyloxy-milbemycin $A_4$,
13β-N,N-Dimethylcarbamoyloxy-milbemycin D,
13β-N,N-Dimethylcarbamoyloxy-milbemycin $A_3$,
13β-N,N-Dimethylcarbamoyloxy-milbemycin $A_4$,
13β-N,N-Diisopropylcarbamoyloxy-milbemycin D,
13β-N,N-Diisopropylcarbamoyloxy-milbemycin $A_3$ und
13β-N,N-Diisopropylcarbamoyloxy-milbemycin $A_4$.

11. Mittel nach Anspruch 2, enthaltend als Wirkstoff eine Verbindung der Formel I, ausgewählt aus der Reihe:
5-O-tert.-Butyldimethylsilyl-13β-N-methylylcarbamoyloxy-milbemycin D,
5-O-tert.-Butyldimethylsilyl-13β-N-methylylcarbamoyloxy-milbemycin $A_3$,
5-O-tert.-Butyldimethylsilyl-13β-N-methylylcarbamoyloxy-milbemycin $A_4$,
5-O-tert.-Butyldimethylsilyl-13β-N-tert.-butylcarbamoyloxy-milbemycin D,
5-O-tert.-Butyldimethylsilyl-13β-N-tert.-butylcarbamoyloxy-milbemycin $A_3$,
5-O-tert.-Butyldimethylsilyl-13β-N-tert.-butylcarbamoyloxy-milbemycin $A_4$,
5-O-tert.-Butyldimethylsilyl-13β-N-phenylcarbamoyloxy-milbemycin D,
5-O-tert.-Butyldimethylsilyl-13β-N-phenylcarbamoyloxy-milbemycin $A_3$,
5-O-tert.-Butyldimethylsilyl-13β-N-phenylcarbamoyloxy-milbemycin $A_4$,
5-O-tert.-Butyldimethylsilyl-13β-N,N-dimethylcarbamoyloxy-milbemycin D,
5-O-tert.-Butyldimethylsilyl-13β-N,N-dimethylcarbamoyloxy-milbemycin $A_3$,
5-O-tert.-Butyldimethylsilyl-13β-N,N-dimethylcarbamoyloxy-milbemycin $A_4$,
5-O-tert.-Butyldimethylsilyl-13β-N,N-diisopropylcarbamoyloxy-milbemycin D,
5-O-tert.-Butyldimethylsilyl-13β-N,N-diisopropylcarbamoyloxy-milbemycin $A_3$ und
5-O-tert.-Butyldimethylsilyl-13β-N,N-diisopropylcarbamoyloxy-milbemycin $A_4$.

12. Verwendung einer Verbindung der Formel I

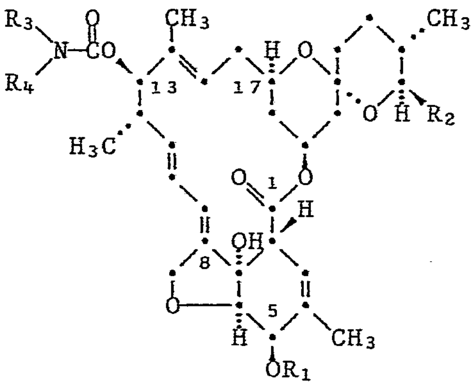

(I)

in welcher
$R_1$ Wasserstoff, eine Silyl- oder Acylgruppe,
$R_2$ Methyl, Ethyl, Isopropyl oder sek.Butyl,
$R_3$ eine unsubstituierte oder substituierte Gruppe ausgewählt aus der Reihe geradkettiges oder verzweigtes $C_1$-$C_{18}$-Alkyl, $C_3$-$C_{10}$-Cycloalkyl, $C_2$-$C_6$-Alkenyl, $C_3$-$C_{10}$-Cycloalkenyl, Phenyl und Naphthyl, und
$R_4$ Wasserstoff oder einen unter $R_3$ angegebenen Rest bedeuten, zur Bekämpfung von Insekten, von Schädlingen der Ordnung Acarina, oder von Nematoden.

13. Verwendung nach Anspruch 12, dadurch gekennzeichnet, dass man eine Verbindung der Formel I nach einem der Ansprüche 2 bis 10 einsetzt.

14. Verwendung nach einem der Ansprüche 12 oder 13, dadurch gekennzeichnet, dass es sich bei den Schädlingen um Ekto- oder Endoparasiten bei Nutztieren handelt.

15. Verfahren zur Bekämpfung von tierparasitären oder pflanzenparasitären Schädlingen, dadurch gekennzeichnet, dass man eine Verbindung der Formel I

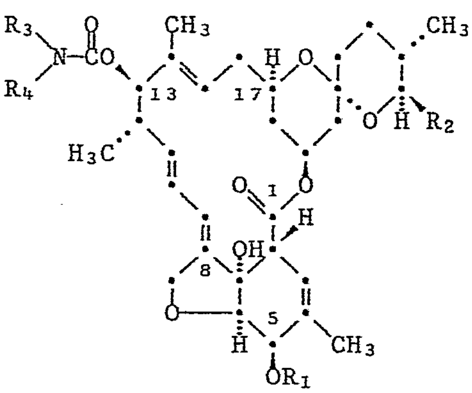

(I)

in welcher
$R_1$ Wasserstoff, eine Silyl- oder Acylgruppe,
$R_2$ Methyl, Ethyl, Isopropyl oder sek.Butyl,
$R_3$ eine unsubstituierte oder substituierte Gruppe ausgewählt aus der Reihe geradkettiges oder verzweigtes $C_1$-$C_{18}$-Alkyl, $C_3$-$C_{10}$-Cycloalkyl, $C_2$-$C_6$-Alkenyl, $C_3$-$C_{10}$-Cycloalkenyl, Phenyl und Naphthyl, und
$R_4$ Wasserstoff oder einen unter $R_3$ angegebenen Rest bedeuten, auf den Parasiten, das Wirtstier, die Pflanze oder den Lebensraum des Parasiten appliziert.

16. Verfahren nach Anspruch 15, dadurch gekennzeichnet, dass man bei dem Verfahren eine Verbindung der Formel I gemäss einem der Ansprüche 3 bis 11 einsetzt.

Patentansprüche für den folgenden Vertragsstaat: ES

1. Verfahren zur Herstellung von Verbindung der Formel I

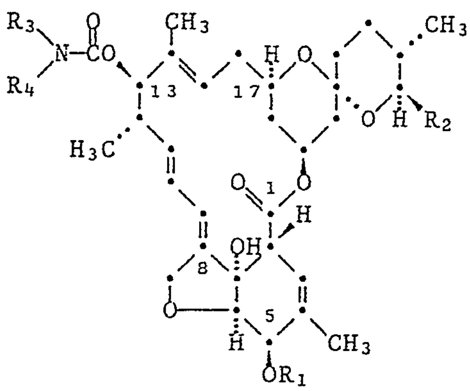

(I)

in welcher

$R_1$ Wasserstoff, eine Silyl- oder Acylgruppe,

$R_2$ Methyl, Ethyl, Isopropyl oder sek.Butyl,

$R_3$ eine unsubstituierte oder substituierte Gruppe ausgewählt aus der Reihe geradkettiges oder verzweigtes $C_1$-$C_{18}$-Alkyl, $C_3$-$C_{10}$-Cycloalkyl, $C_2$-$C_6$-Alkenyl, $C_3$-$C_{10}$-Cycloalkenyl, Phenyl und Naphthyl, und

$R_4$ Wasserstoff oder einen unter $R_3$ angegebenen Rest bedeuten, dadurch gekennzeichnet, dass man

A) in den Fällen, in denen eine Verbindung der Formel I hergestellt werden soll, worin $R_4$ für Wasserstoff steht, wird ein Allylalkohol der Formel II

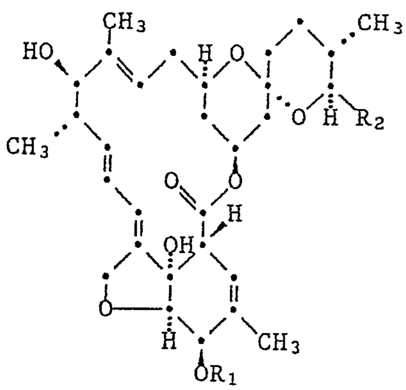

(II),

worin $R_1$ eine Schutzgruppe bedeutet und $R_2$ die unter Formel I angegebene Bedeutung besitzt, mit einem zur Einführung einer 13β-Carbamoyloxygruppe $R_3NH$-C(O)O- geeigneten Reagenz behandelt und danach, sofern ein Produkt mit einer freien 5-Hydroxy-Gruppe erwünscht ist, die Schutzgruppe $R_1$ hydrolytisch abgespaltet, oder

B) in den Fällen, in denen eine Verbindung der Formel I hergestellt werden soll, worin $R_4$ eine der unter $R_3$ angegebenen Bedeutungen hat, wird ein Allylalkohol der Formel II, worin $R_1$ eine Schutzgruppe bedeutet und $R_2$ die unter Formel I angegebene Bedeutung besitzt, mit einem zur Einführung einer 13β-Carbamoyloxygruppe

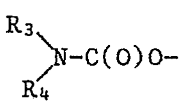

($R_4$ $\neq$ H) geeigneten Reagenz behandelt und danach, sofern ein Produkt mit einer freien 5-Hydroxy-Gruppe erwünscht ist, die Schutzgruppe $R_1$ hydrolytisch abgespaltet, oder

C) durch Umsetzung einer Verbindung der Formel V,

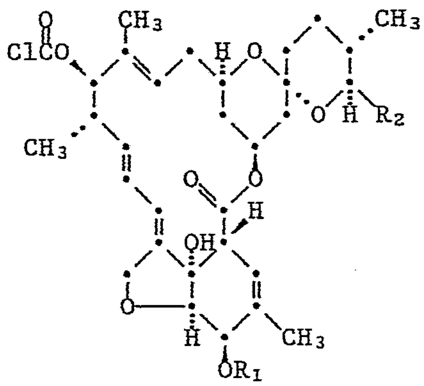

(V),

worin $R_1$ für eine Schutzgruppe steht und $R_2$ eine der unter Formel I angegebenen Bedeutungen hat, mit einem primären oder sekundären Amin der Formel VI,

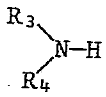

(VI),

worin $R_3$ und $R_4$ wie unter Formel I definiert sind, erhalten werden und sofern ein Produkt mit einer freien 5-Hydroxy-Gruppe erwünscht ist, durch anschliessende hydrolytische Abspaltung der Schutzgruppe $R_1$,

2. Pestizides Mittel dadurch gekennzeichnet, dass es neben üblichen Formulierungshilfsstoffen als Wirkkomponente mindestens eine Verbindung der Formel I

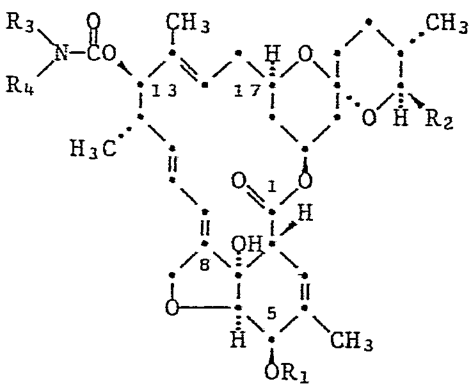

(I)

in welcher
$R_1$ Wasserstoff, eine Silyl- oder Acylgruppe,
$R_2$ Methyl, Ethyl, Isopropyl oder sek.Butyl,
$R_3$ eine unsubstituierte oder substituierte Gruppe ausgewählt aus der Reihe geradkettiges oder verzweigtes $C_1$-$C_{18}$-Alkyl, $C_3$-$C_{10}$-Cycloalkyl, $C_2$-$C_6$-Alkenyl, $C_3$-$C_{10}$-Cycloalkenyl, Phenyl und Naphthyl, und
$R_4$ Wasserstoff oder einen unter $R_3$ angegebenen Rest bedeuten, enthält.

3. Mittel nach Anspruch 2, enthaltend als Wirkstoff eine der Verbindungen der Formel I, worin
$R_1$ Wasserstoff darstellt,
$R_2$ für Methyl, Ethyl, Isopropyl oder sek.Butyl steht,
$R_3$ unsubstituiertes oder durch Halogen, $C_1$-$C_4$-Alkoxy, Acyloxy oder eine unsubstituierte oder durch Halogen, $C_1$-$C_2$-Alkyl, $C_1$-$C_2$-Alkoxy, $C_1$-$C_2$-Haloalkyl oder Nitro substituierte Phenyl- oder Naphthylgruppe substituiertes $C_1$-$C_{18}$-Alkyl, unsubstituiertes oder durch Halogen und/oder $C_1$-$C_4$-Alkyl substituiertes $C_3$-$C_{10}$-Cycloalkyl, unsubstituiertes oder durch Halogen substituiertes $C_2$-$C_6$-Alkenyl, unsubstituiertes oder durch Halogen und/oder $C_1$-$C_4$-Alkyl substituiertes $C_3$-$C_{10}$-Cycloalkenyl darstellt, und
$R_4$ für Wasserstoff steht.

4. Mittel nach Anspruch 3, enthaltend als Wirkstoff eine der Verbindungen der Formel I, worin
$R_1$ Wasserstoff darstellt,
$R_2$ für Methyl, Ethyl, Isopropyl oder sek.Butyl steht, und
$R_3$ unsubstituiertes oder durch Halogen, Phenyl oder Naphthyl substituiertes $C_1$-$C_8$-Alkyl, $C_5$-$C_7$-Cycloal-

kyl, $C_2$-$C_6$-Alkenyl und $C_5$-$C_7$-Cycloalkenyl darstellt, und
$R_4$ für Wasserstoff steht.

5. Mittel nach Anspruch 3, enthaltend als Wirkstoff eine der Verbindungen der Formel I, worin
$R_1$ Wasserstoff darstellt,
$R_2$ für Methyl, Aethyl, Isopropyl oder sek.Butyl steht, und
$R_3$ unsubstituiertes oder durch Halogen, $C_1$-$C_6$-Alkyl, $C_1$-$C_6$-Alkoxy, $C_1$-$C_4$-Alkylthio, $C_1$-$C_4$-Haloalkyl oder Nitro substituiertes Phenyl oder Naphthyl darstellt, und
$R_4$ für Wasserstoff steht.

6. Mittel nach Anspruch 5, enthaltend als Wirkstoff eine der Verbindungen der Formel I, worin
$R_1$ Wasserstoff darstellt,
$R_2$ für Methyl, Aethyl, Isopropyl oder sek.Butyl steht, und
$R_3$ unsubstituiertes oder durch Brom, Chlor, Fluor, $C_1$-$C_2$-Alkyl, $C_1$-$C_2$-Alkoxy, $C_1$-$C_2$-Haloalkyl oder Nitro substituiertes Phenyl oder Naphthyl darstellt, und
$R_4$ für Wasserstoff steht.

7. Mittel nach Anspruch 6, enthaltend als Wirkstoff eine der Verbindungen der Formel I, worin
$R_1$ Wasserstoff darstellt,
$R_2$ für Methyl, Ethyl, Isopropyl oder sek. Butyl steht, und
$R_3$ unsubstituiertes oder durch Brom, Chlor, Fluor, $C_1$-$C_2$-Alkyl, $C_1$-$C_2$-Alkoxy, Trifluormethyl oder Nitro substituiertes Phenyl oder unsubstituiertes Naphthyl darstellt.

8. Mittel nach Anspruch 3, enthaltend als Wirkstoff eine der Verbindungen der Formel I, worin
$R_1$ Wasserstoff darstellt,
$R_2$ für Methyl, Ethyl, Isopropyl oder sek.Butyl steht, und
$R_3$ unsubstituiertes oder durch Halogen, $C_1$-$C_4$-Alkoxy, oder $C_1$-$C_4$-Acyloxy substituiertes $C_1$-$C_8$-Alkyl, unsubstituiertes oder durch Halogen, $C_1$-$C_6$-Alkyl, $C_1$-$C_4$-Alkylthio, $C_1$-$C_4$-Haloalkyl oder Nitro substituiertes Phenyl darstellt, und
$R_4$ einen unter $R_3$ angegebenen Rest bedeutet.

9. Mittel nach Anspruch 8, enthaltend als Wirkstoff eine der Verbindungen der Formel I, worin
$R_1$ Wasserstoff darstellt,
$R_2$ für Methyl, Ethyl, Isopropyl oder sek.Butyl steht, und
$R_3$ $C_1$-$C_8$-Alkyl, oder Phenyl darstellt, und
$R_4$ einen unter $R_3$ angegebenen Rest bedeutet.

10. Mittel nach Anspruch 2, enthaltend als Wirkstoff eine Verbindung der Formel I, ausgewählt aus der Reihe:
13β-N-Methylcarbamoyloxy-milbemycin D,
13β-N-Methylcarbamoyloxy-milbemycin $A_3$,
13β-N-Methylcarbamoyloxy-milbemycin $A_4$,
13β-N-tert.-Butylcarbamoyloxy-milbemycin D,
13β-N-tert.-Butylcarbamoyloxy-milbemycin $A_3$,
13β-N-tert.-Butylcarbamoyloxy-milbemycin $A_4$,
13β-N-Phenylcarbamoyloxy-milbemycin D,
13β-N-Phenylcarbamoyloxy-milbemycin $A_3$,
13β-N-Phenylcarbamoyloxy-milbemycin $A_4$,
13β-N,N-Dimethylcarbamoyloxy-milbemycin D,
13β-N,N-Dimethylcarbamoyloxy-milbemycin $A_3$,
13β-N,N-Dimethylcarbamoyloxy-milbemycin $A_4$,
13β-N,N-Diisopropylcarbamoyloxy-milbemycin D,
13β-N,N-Diisopropylcarbamoyloxy-milbemycin $A_3$ und
13β-N,N-Diisopropylcarbamoyloxy-milbemycin $A_4$.

11. Mittel nach Anspruch 2, enthaltend als Wirkstoff eine Verbindung der Formel I, ausgewählt aus der Reihe:
5-O-tert.-Butyldimethylsilyl-13β-N-methylylcarbamoyloxy-milbemycin D,
5-O-tert.-Butyldimethylsilyl-13β-N-methylylcarbamoyloxy-milbemycin $A_3$,
5-O-tert.-Butyldimethylsilyl-13β-N-methylylcarbamoyloxy-milbemycin $A_4$,
5-O-tert.-Butyldimethylsilyl-13β-N-tert.-butylcarbamoyloxy-milbemycin D,
5-O-tert.-Butyldimethylsilyl-13β-N-tert.-butylcarbamoyloxy-milbemycin $A_3$,
5-O-tert.-Butyldimethylsilyl-13β-N-tert.-butylcarbamoyloxy-milbemycin $A_4$,
5-O-tert.-Butyldimethylsilyl-13β-N-phenylcarbamoyloxy-milbemycin D,
5-O-tert.-Butyldimethylsilyl-13β-N-phenylcarbamoyloxy-milbemycin $A_3$,
5-O-tert.-Butyldimethylsilyl-13β-N-phenylcarbamoyloxy-milbemycin $A_4$,
5-O-tert.-Butyldimethylsilyl-13β-N,N-dimethylcarbamoyloxy-milbemycin D,
5-O-tert.-Butyldimethylsilyl-13β-N,N-dimethylcarbamoyloxy-milbemycin $A_3$,
5-O-tert.-Butyldimethylsilyl-13β-N,N-dimethylcarbamoyloxy-milbemycin $A_4$,
5-O-tert.-Butyldimethylsilyl-13β-N,N-diisopropylcarbamoyloxy-milbemycin D,
5-O-tert.-Butyldimethylsilyl-13β-N,N-diisopropylcarbamoyloxy-milbemycin $A_3$ und
5-O-tert.-Butyldimethylsilyl-13β-N,N-diisopropylcarbamoyloxy-milbemycin $A_4$.

Europäisches Patentamt

# EUROPÄISCHER RECHERCHENBERICHT

Nummer der Anmeldung
EP 87 81 0109

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 008 184 (MERCK & CO. INC.)<br>* Seiten 5-6; Seiten 28-31 *<br>--- | 1,12 | C 07 D 493/22<br>C 07 F 7/04<br>A 61 K 31/35 //<br>(C 07 D 493/22<br>C 07 D 313:00<br>C 07 D 311:00<br>C 07 D 311:00<br>C 07 D 307:00 ) |
| P,A | EP-A-0 184 308 (SANKYO)<br>* Seite 5 *<br>----- | 1 | |
| | | | **RECHERCHIERTE SACHGEBIETE (Int. Cl.4)** |
| | | | C 07 D 493/00<br>A 61 K 31/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 29-06-1987 | VERHULST W. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82